# EUROPEAN PATENT APPLICATION

(11) **EP 4 059 963 A1**
(43) Date of publication of application: **21.09.2022**
(21) Application number: 20886450.4
(22) Date of filing: 11.11.2020
(51) Int. Cl.: C07K 16/28, C12N 15/13, C12N 1/00, C12N 5/10, C12N 15/63

(54) **MOLECULE CAPABLE OF BINDING TO HUMAN 4-1BB, AND APPLICATION OF MOLECULE**

(30) Priority: 13.11.2019 CN 201911105611; 13.11.2019 CN 201911105605; 13.11.2019 CN 201911106016
(71) Applicant: Hefei Hankemab Biotechnology Co., Ltd, Hefei, Anhu 230088 (CN)
(72) Inventor: CHENG, Liansheng, Hefei, Anhui 230088 (CN); LIU, Wenting, Hefei, Anhui 230088 (CN); ZHANG, Dayan, Hefei, Anhui 230088 (CN); ZENG, Xiaoli, Hefei, Anhui 230088 (CN); WANG, Fengrong, Hefei, Anhui 230088 (CN)
(74) Representative: Isarpatent
(86) International application number: PCT/CN2020/127993
(87) International publication number: WO 2021/093753

(57) **Abstract**

The present invention discloses a molecule capable of binding to human 4-1BB and its application thereof. The amino acid sequences of HCDR1, HCDR2 and HCDR3 in the heavy chain variable region of the antibody provided by present invention are shown sequentially at positions 31-35, positions 50-64, and positions 98-106 in SEQ ID No.1 from the N-terminus. The amino acid sequences of LCDR1, LCDR2 and LHCDR3 in the light chain variable region are shown sequentially at positions 24-34, positions 50-56, and positions 89-97 in SEQ ID No.2 from the N-terminus. The antibodies provided by the present invention can bind to human and monkey 4-1BB, exhibit high affinity to human 4-1BB and effectively enhance T cell responses; they can be used to regulate the immune responses mediated by T cells and antibodies; as immune modulators, they have a wide range of therapeutic uses in diseases such as cancer, autoimmune diseases, inflammatory diseases, and infectious diseases, etc.

<110>  Hefei HankeMab Biotechnology Co., Ltd
<120>  MOLECULE CAPABLE OF BINDING TO HUMAN 4-1BB, AND APPLICATION OF MOLECULE
<130>  P54382-WOEP
<140>  20886450.4
<141>  2020-11-11
<150>  201911105611.1
<151>  2019-11-13
<150>  201911105605.6
<151>  2019-11-13
<150>  201911106016.X
<151>  2019-11-13
<160>  28
<170>  PatentIn version 3.5

<210>  1
<211>  444
<212>  PRT
<213>  Artificial sequence
<220>
<223>  Synthesized
<400>  1
Gln Val Gln Leu Gln Glu Ser Gly Pro Gly Leu Val Lys Pro Ser Glu
1               5                   10                  15

Thr Leu Ser Ile Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Leu Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys

130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Glu Ser Lys Tyr Gly Pro Pro Cys Pro
    210                 215                 220

Pro Cys Pro Ala Pro Glu Phe Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser Gln Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Gly Leu Pro Ser Ser Ile Glu Lys Thr Ile Ser Lys Ala
                325                 330                 335

Lys Gly Gln Pro Arg Glu Pro Gln Val Tyr Thr Leu Pro Pro Ser Gln
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Arg Leu Thr Val Asp Lys Ser Arg Trp Gln Glu
                405                 410                 415

Gly Asn Val Phe Ser Cys Ser Val Met His Glu Ala Leu His Asn His
            420                 425                 430

Tyr Thr Gln Lys Ser Leu Ser Leu Ser Leu Gly Lys
        435                 440

<210>  2
<211>  214
<212>  PRT
<213>  Artificial sequence
<220>
<223>  Synthesized
<400>  2
Asp Ile Gln Met Thr Gln Ser Pro Ser Ser Leu Ser Ala Ser Leu Gly
1               5                   10                  15

Asp Arg Val Thr Ile Ser Cys Ser Ala Ser Gln Gly Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Thr Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Gln Tyr Ser Lys Leu Pro Trp
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  3
<211>  1332
<212>  DNA
<213>  Artificial sequence
<220>
<223>  Synthesized
<400>  3
caggtgcagc tgcaggagtc tggcccaggc ctggtgaagc cctctgagac cctgagcatc      60

acctgtacag tgagcggcag ctccctgaca tcctacggag tgcactgggt gcggcagcca     120

cctggcaagg gcctggaggg actgggcgtg atctggccag gaggcagcac caactataat     180

tccgccctga tgagccggct gacaatctct aaggacaaca gcaagtccca ggtgagcctg     240

aagatgtcta gcctgaccgc cgccgacaca gccatgtact attgcgcccg ggtgaccggc     300

acatggtact tcgacgtgtg gggccagggc accacagtga ccgtgtcctc tgctagcacc     360

aagggcccat ccgtcttccc cctggcgccc tgctccagga gcacctccga gagcacagcc     420

gccctgggct gcctggtcaa ggactacttc cccgaaccgg tgacggtgtc gtggaactca 480

ggcgccctga ccagcggcgt gcacaccttc ccggctgtcc tacagtcctc aggactctac 540

tccctcagca gcgtggtgac cgtgccctcc agcagcttgg gcacgaagac ctacacctgc 600

aacgtagatc acaagcccag caacaccaag gtggacaaga gagttgagtc caaatatggt 660

cccccatgcc caccatgccc agcacctgag ttcctggggg gaccatcagt cttcctgttc 720

cccccaaaac ccaaggacac tctcatgatc tcccggaccc ctgaggtcac gtgcgtggtg 780

gtggacgtga gccaggaaga ccccgaggtc cagttcaact ggtacgtgga tggcgtggag 840

gtgcataatg ccaagacaaa gccgcgggag gagcagttca acagcacgta ccgtgtggtc 900

agcgtcctca ccgtcctgca ccaggactgg ctgaacggca aggagtacaa gtgcaaggtc 960

tccaacaaag gcctcccgtc ctccatcgag aaaaccatct ccaaagccaa agggcagccc 1020

cgagagccac aggtgtacac cctgccccca tcccaggagg agatgaccaa gaaccaggtc 1080

agcctgacct gcctggtcaa aggcttctac cccagcgaca tcgccgtgga gtgggagagc 1140

aatgggcagc cggagaacaa ctacaagacc acgcctcccg tgctggactc cgacggctcc 1200

ttcttcctct acagcagact aaccgtggac aagagcaggt ggcaggaggg gaatgtcttc 1260

tcatgctccg tgatgcatga ggctctgcac aaccactaca cacagaagag cctctccctg 1320

tctctgggta aa 1332

<210> 4
<211> 642
<212> DNA
<213> Artificial sequence
<220>
<223> Synthesized
<400> 4
gacatccaga tgacccagtc cccaagctcc ctgtccgcct ctctgggcga tagggtgaca 60

atcagctgct ccgcctctca gggcatctcc aactacctga attggtatca gcagaagcca 120

gacggcaccg tgaagctgct gatctactat accagcacac tgcactccgg agtgccaagc 180

cggttcagcg gctccggctc tggcaccgac tttaccctga caatctctag cctgcagccc 240

gaggatgtgg ccacatacta ttgtcagcag tactctaagc tgccttggac cttcggcggc 300

ggcacaaagc tggagatcaa gcgtacggtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg          540

ctgagcaaag cagactacga gaaacacaaa gtctacgcct gcgaagtcac ccatcagggc          600

ctgagctcgc ccgtcacaaa gagcttcaac aggggagagt gc                              642

<210>  5
<211>  255
<212>  PRT
<213>  Artificial sequence
<220>
<223>  Synthesized
<400>  5
Met Gly Asn Ser Cys Tyr Asn Ile Val Ala Thr Leu Leu Leu Val Leu
1               5                   10                  15

Asn Phe Glu Arg Thr Arg Ser Leu Gln Asp Pro Cys Ser Asn Cys Pro
            20                  25                  30

Ala Gly Thr Phe Cys Asp Asn Asn Arg Asn Gln Ile Cys Ser Pro Cys
        35                  40                  45

Pro Pro Asn Ser Phe Ser Ser Ala Gly Gly Gln Arg Thr Cys Asp Ile
    50                  55                  60

Cys Arg Gln Cys Lys Gly Val Phe Arg Thr Arg Lys Glu Cys Ser Ser
65                  70                  75                  80

Thr Ser Asn Ala Glu Cys Asp Cys Thr Pro Gly Phe His Cys Leu Gly
                85                  90                  95

Ala Gly Cys Ser Met Cys Glu Gln Asp Cys Lys Gln Gly Gln Glu Leu
            100                 105                 110

Thr Lys Lys Gly Cys Lys Asp Cys Cys Phe Gly Thr Phe Asn Asp Gln
        115                 120                 125

Lys Arg Gly Ile Cys Arg Pro Trp Thr Asn Cys Ser Leu Asp Gly Lys
    130                 135                 140

Ser Val Leu Val Asn Gly Thr Lys Glu Arg Asp Val Val Cys Gly Pro
145                 150                 155                 160

Ser Pro Ala Asp Leu Ser Pro Gly Ala Ser Ser Val Thr Pro Pro Ala
                165                 170                 175

Pro Ala Arg Glu Pro Gly His Ser Pro Gln Ile Ile Ser Phe Phe Leu
            180                 185                 190

Ala Leu Thr Ser Thr Ala Leu Leu Phe Leu Leu Phe Phe Leu Thr Leu
        195                 200                 205

Arg Phe Ser Val Val Lys Arg Gly Arg Lys Lys Leu Leu Tyr Ile Phe
    210                 215                 220

Lys Gln Pro Phe Met Arg Pro Val Gln Thr Thr Gln Glu Glu Asp Gly
225                 230                 235                 240

Cys Ser Cys Arg Phe Pro Glu Glu Glu Glu Gly Gly Cys Glu Leu
                245                 250                 255

<210>  6
<211>  254
<212>  PRT
<213>  Artificial sequence
<220>
<223>  Synthesized
<400>  6
Met Gly Asn Ser Cys Tyr Asn Ile Val Ala Thr Leu Leu Leu Val Leu
1               5                   10                  15

Asn Phe Glu Arg Thr Arg Ser Leu Gln Asp Leu Cys Ser Asn Cys Pro
            20                  25                  30

Ala Gly Thr Phe Cys Asp Asn Asn Arg Ser Gln Ile Cys Ser Pro Cys
        35                  40                  45

Pro Pro Asn Ser Phe Ser Ser Ala Gly Gly Gln Arg Thr Cys Asp Ile
    50                  55                  60

Cys Arg Gln Cys Lys Gly Val Phe Lys Thr Arg Lys Glu Cys Ser Ser
65                  70                  75                  80

Thr Ser Asn Ala Glu Cys Asp Cys Ile Ser Gly Tyr His Cys Leu Gly
                85                  90                  95

Ala Glu Cys Ser Met Cys Glu Gln Asp Cys Lys Gln Gly Gln Glu Leu
            100                 105                 110

Thr Lys Lys Gly Cys Lys Asp Cys Cys Phe Gly Thr Phe Asn Asp Gln
        115                 120                 125

Lys Arg Gly Ile Cys Arg Pro Trp Thr Asn Cys Ser Leu Asp Gly Lys
    130                 135                 140

Ser Val Leu Val Asn Gly Thr Lys Glu Arg Asp Val Val Cys Gly Pro
145                 150                 155                 160

Ser Pro Ala Asp Leu Ser Pro Gly Ala Ser Ser Ala Thr Pro Pro Ala
                165                 170                 175

Pro Ala Arg Glu Pro Gly His Ser Pro Gln Ile Ile Phe Phe Leu Ala
            180                 185                 190

Leu Thr Ser Thr Val Val Leu Phe Leu Leu Phe Phe Leu Val Leu Arg
        195                 200                 205

Phe Ser Val Val Lys Arg Ser Arg Lys Lys Leu Leu Tyr Ile Phe Lys
    210                 215                 220

Gln Pro Phe Met Arg Pro Val Gln Thr Thr Gln Glu Glu Asp Gly Cys
225                 230                 235                 240

Ser Cys Arg Phe Pro Glu Glu Glu Glu Gly Gly Cys Glu Leu
                245                 250

<210>  7
<211>  765
<212>  DNA
<213>  Artificial sequence
<220>
<223>  Synthesized
<400>  7
atgggcaact cctgctacaa catcgtggcc accctgctgc tggtgctgaa cttcgagcgc      60

acccgctccc tgcaggaccc atgctccaat tgtcccgccg gcaccttctg cgataacaat     120

aggaaccaga tctgctctcc ctgtccccct aatagcttta gctccgccgg aggacagagg     180

acatgcgaca tctgtagaca gtgcaagggc gtgttcagga cccgcaagga gtgttctagc 240

acaagcaacg ccgagtgcga ctgtacccct ggatttcact gcctgggagc aggatgttcc 300

atgtgcgagc aggattgtaa gcagggccag gagctgacca agaagggctg caaggactgc 360

tgtttcggca ccttcaacga tcagaagagg ggcatctgtc gcccttggac caactgcagc 420

ctggatggca agtccgtgct ggtgaatggc acaaaggaga gggacgtggt gtgcggacct 480

tctccagccg atctgagccc aggcgcctcc tctgtgaccc caccagcacc agcaagagag 540

cctggacact ccccacagat catctccttc tttctggccc tgacctctac agccctgctg 600

ttcctgctgt tctttctgac cctgaggttt tccgtggtga agaggggccg caagaagctg 660

ctgtacatct tcaagcagcc cttcatgaga cccgtgcaga ccacacagga ggaggatggc 720

tgctcttgta ggtttccaga ggaggaggag ggaggatgtg agctg 765

<210> 8
<211> 762
<212> DNA
<213> Artificial sequence
<220>
<223> Synthesized
<400> 8
atgggcaact cctgttacaa tatcgtggcc accctgctgc tggtgctgaa cttcgagcgg 60

acaagaagcc tgcaggacct gtgctccaat tgtcctgccg gcaccttctg cgataacaat 120

cggagccaga tctgctcccc atgtccccct aactctttta gctccgccgg aggacagagg 180

acatgcgaca tctgtagaca gtgcaagggc gtgtttaaga cccggaagga gtgttctagc 240

acatctaatg ccgagtgcga ctgtatcagc ggctatcact gcctgggcgc cgagtgtagc 300

atgtgcgagc aggattgtaa gcagggccag gagctgacca agaagggctg caaggactgc 360

tgtttcggca ccttcaacga tcagaagagg ggcatctgtc gcccctggac caactgctct 420

ctggatggca agagcgtgct ggtgaatggc acaaaggaga gagacgtggt gtgcggccct 480

tccccagcag atctgtctcc tggcgcctcc tctgccaccc cacccgcccc agccagggag 540

cccggccact ccccccagat catcttcttt ctggccctga ccagcacagt ggtgctgttc 600

ctgctgttct ttctggtgct gcggtttagc gtggtgaagc ggtccagaaa gaagctgctg 660

tacatcttca agcagccttt tatgaggcca gtgcagacca cacaggagga ggacggctgc 720

tcctgtaggt tcccagagga ggaggaggga ggatgcgagc tg 762

<210>  9
<211>  254
<212>  PRT
<213>  Artificial sequence
<220>
<223>  Synthesized
<400>  9
Met Glu Tyr Ala Ser Asp Ala Ser Leu Asp Pro Glu Ala Pro Trp Pro
1               5                   10                  15

Pro Ala Pro Arg Ala Arg Ala Cys Arg Val Leu Pro Trp Ala Leu Val
            20                  25                  30

Ala Gly Leu Leu Leu Leu Leu Leu Leu Ala Ala Ala Cys Ala Val Phe
        35                  40                  45

Leu Ala Cys Pro Trp Ala Val Ser Gly Ala Arg Ala Ser Pro Gly Ser
    50                  55                  60

Ala Ala Ser Pro Arg Leu Arg Glu Gly Pro Glu Leu Ser Pro Asp Asp
65                  70                  75                  80

Pro Ala Gly Leu Leu Asp Leu Arg Gln Gly Met Phe Ala Gln Leu Val
                85                  90                  95

Ala Gln Asn Val Leu Leu Ile Asp Gly Pro Leu Ser Trp Tyr Ser Asp
            100                 105                 110

Pro Gly Leu Ala Gly Val Ser Leu Thr Gly Gly Leu Ser Tyr Lys Glu
        115                 120                 125

Asp Thr Lys Glu Leu Val Val Ala Lys Ala Gly Val Tyr Tyr Val Phe
    130                 135                 140

Phe Gln Leu Glu Leu Arg Arg Val Val Ala Gly Glu Gly Ser Gly Ser
145                 150                 155                 160

Val Ser Leu Ala Leu His Leu Gln Pro Leu Arg Ser Ala Ala Gly Ala
                165                 170                 175

Ala Ala Leu Ala Leu Thr Val Asp Leu Pro Pro Ala Ser Ser Glu Ala
            180                 185                 190

Arg Asn Ser Ala Phe Gly Phe Gln Gly Arg Leu Leu His Leu Ser Ala
        195                 200                 205

Gly Gln Arg Leu Gly Val His Leu His Thr Glu Ala Arg Ala Arg His
    210                 215                 220

Ala Trp Gln Leu Thr Gln Gly Ala Thr Val Leu Gly Leu Phe Arg Val
225                 230                 235                 240

Thr Pro Glu Ile Pro Ala Gly Leu Pro Ser Pro Arg Ser Glu
                245                 250

<210> 10
<211> 256
<212> PRT
<213> Artificial sequence
<220>
<223> Synthesized
<400> 10
Met Gly Asn Asn Cys Tyr Asn Val Val Val Ile Val Leu Leu Leu Val
1               5                   10                  15

Gly Cys Glu Lys Val Gly Ala Val Gln Asn Ser Cys Asp Asn Cys Gln
            20                  25                  30

Pro Gly Thr Phe Cys Arg Lys Tyr Asn Pro Val Cys Lys Ser Cys Pro
        35                  40                  45

Pro Ser Thr Phe Ser Ser Ile Gly Gly Gln Pro Asn Cys Asn Ile Cys
    50                  55                  60

Arg Val Cys Ala Gly Tyr Phe Arg Phe Lys Lys Phe Cys Ser Ser Thr
65                  70                  75                  80

His Asn Ala Glu Cys Glu Cys Ile Glu Gly Phe His Cys Leu Gly Pro
                85                  90                  95

Gln Cys Thr Arg Cys Glu Lys Asp Cys Arg Pro Gly Gln Glu Leu Thr
            100                 105                 110

Lys Gln Gly Cys Lys Thr Cys Ser Leu Gly Thr Phe Asn Asp Gln Asn
        115                 120                 125

Gly Thr Gly Val Cys Arg Pro Trp Thr Asn Cys Ser Leu Asp Gly Arg
    130                 135                 140

Ser Val Leu Lys Thr Gly Thr Thr Glu Lys Asp Val Val Cys Gly Pro
145                 150                 155                 160

Pro Val Val Ser Phe Ser Pro Ser Thr Thr Ile Ser Val Thr Pro Glu
                165                 170                 175

Gly Gly Pro Gly Gly His Ser Leu Gln Val Leu Thr Leu Phe Leu Ala
            180                 185                 190

Leu Thr Ser Ala Leu Leu Leu Ala Leu Ile Phe Ile Thr Leu Leu Phe
        195                 200                 205

Ser Val Leu Lys Trp Ile Arg Lys Lys Phe Pro His Ile Phe Lys Gln
    210                 215                 220

Pro Phe Lys Lys Thr Thr Gly Ala Ala Gln Glu Glu Asp Ala Cys Ser
225                 230                 235                 240

Cys Arg Cys Pro Gln Glu Glu Glu Gly Gly Gly Gly Gly Tyr Glu Leu
                245                 250                 255

<210>  11
<211>  768
<212>  DNA
<213>  Artificial sequence
<220>
<223>  Synthesized
<400>  11
atgggcaaca attgttacaa cgtggtggtc atcgtgctgc tgctggtggg atgcgagaaa      60

gtgggagccg tgcagaactc ctgcgacaat tgtcagcccg gcaccttctg taggaagtac     120

aatcccgtgt gcaagtcctg tccccctagc accttcagct ccatcggcgg ccagcccaac     180

tgcaatatct gtagggtgtg cgccggctat ttccgcttta agaagttctg ctctagcacc     240

cacaacgccg agtgcgagtg tatcgagggc tttcactgtc tgggacccca gtgtaccagg     300

tgcgagaagg actgcagacc aggacaggag ctgacaaagc agggctgtaa gacctgctct     360

ctgggcacat tcaacgatca gaatggcacc ggcgtgtgcc ggccttggac aaattgcagc     420

ctggacggca gatccgtgct gaagaccggc accacagaga aggatgtggt gtgcggccca 480

cccgtggtgt ccttttctcc tagcaccaca atctctgtga cacctgaggg cggcccagga 540

ggacacagcc tgcaggtgct gaccctgttt ctggccctga caagcgccct gctgctggcc 600

ctgatcttta tcaccctgct gttctccgtg ctgaagtgga tcaggaagaa gttcccacac 660

atctttaagc agcccttcaa gaagaccaca ggagcagcac aggaggagga cgcatgctcc 720

tgtaggtgcc ctcaggaaga agagggagga ggaggaggct acgagctg 768

<210> 12
<211> 14
<212> DNA
<213> Artificial sequence
<220>
<223> Synthesized
<400> 12
tggggacttt ccgc 14

<210> 13
<211> 1650
<212> DNA
<213> Artificial sequence
<220>
<223> Synthesized
<400> 13
atggaagatg ccaaaaacat taagaagggc ccagcgccat tctacccact cgaagacggg 60

accgccggcg agcagctgca caaagccatg aagcgctacg ccctggtgcc cggcaccatc 120

gcctttaccg acgcacatat cgaggtggac attacctacg ccgagtactt cgagatgagc 180

gttcggctgg cagaagctat gaagcgctat gggctgaata caaaccatcg gatcgtggtg 240

tgcagcgaga atagcttgca gttcttcatg cccgtgttgg gtgccctgtt catcggtgtg 300

gctgtggccc cagctaacga catctacaac gagcgcgagc tgctgaacag catgggcatc 360

agccagccca ccgtcgtatt cgtgagcaag aaagggctgc aaaagatcct caacgtgcaa 420

aagaagctac cgatcataca aaagatcatc atcatggata gcaagaccga ctaccagggc 480

ttccaaagca tgtacacctt cgtgacttcc catttgccac ccggcttcaa cgagtacgac 540

ttcgtgcccg agagcttcga ccgggacaaa accatcgccc tgatcatgaa cagtagtggc 600

agtaccggat tgcccaaggg cgtagcccta ccgcaccgca ccgcttgtgt ccgattcagt 660

catgcccgcg accccatctt cggcaaccag atcatccccg acaccgctat cctcagcgtg 720

gtgccatttc accacggctt cggcatgttc accacgctgg gctacttgat ctgcggcttt 780

cgggtcgtgc tcatgtaccg cttcgaggag gagctattct tgcgcagctt gcaagactat 840

aagattcaat ctgccctgct ggtgcccaca ctatttagct tcttcgctaa gagcactctc 900

atcgacaagt acgacctaag caacttgcac gagatcgcca gcggcggggc gccgctcagc 960

aaggaggtag gtgaggccgt ggccaaacgc ttccacctac caggcatccg ccagggctac 1020

ggcctgacag aaacaaccag cgccattctg atcacccccg aaggggacga caagcctggc 1080

gcagtaggca aggtggtgcc cttcttcgag gctaaggtgg tggacttgga caccggtaag 1140

acactgggtg tgaaccagcg cggcgagctg tgcgtccgtg gccccatgat catgagcggc 1200

tacgttaaca accccgaggc tacaaacgct ctcatcgaca aggacggctg gctgcacagc 1260

ggcgacatcg cctactggga cgaggacgag cacttcttca tcgtggaccg gctgaagagc 1320

ctgatcaaat acaagggcta ccaggtagcc ccagccgaac tggagagcat cctgctgcaa 1380

caccccaaca tcttcgacgc cggggtcgcc ggcctgcccg acgacgatgc cggcgagctg 1440

cccgccgcag tcgtcgtgct ggaacacggt aaaaccatga ccgagaagga gatcgtggac 1500

tatgtggcca gccaggttac aaccgccaag aagctgcgcg gtggtgttgt gttcgtggac 1560

gaggtgccta aaggactgac cggcaagttg gacgcccgca agatccgcga gattctcatt 1620

aaggccaaga agggcggcaa gatcgccgtg 1650

<210> 14
<211> 951
<212> DNA
<213> Artificial sequence
<220>
<223> Synthesized
<400> 14
atgaccatgg agacacagat gtcccagaac gtgtgccctc ggaacctgtg gctgctgcag 60

ccactgaccg tgctgctgct gctggcctct gccgatagcc aggcagcagc accacctaag 120

gccgtgctga agctggagcc accctggatc aatgtgctgc aggaggattc tgtgaccctg 180

acatgtcagg gagcacggag ccctgagtct gacagcatcc agtggttcca caatggcaac 240

ctgatcccta cccacacaca gccatcctac cggttcaagg ccaacaataa cgattctggc 300

gagtatacct gccagacagg ccagacctcc ctgtctgacc ccgtgcacct gacagtgctg 360

agcgagtggc tggtgctgca gacccctcac ctggagttcc aggagggcga gacaatcatg 420

ctgcggtgtc actcctggaa ggacaagcca ctggtgaagg tgaccttctt tcagaatggc 480

aagagccaga agttctccca cctggacccc accttcagca tccctcaggc caatcacagc 540

cactccggcg actaccactg cacaggcaac atcggctata ccctgtttag ctccaagccc 600

gtgaccatca cagtgcaggt gccaagcatg ggctctagct cccccatggg catcatcgtg 660

gccgtggtca tcgcaaccgc agtggcagca atcgtggcag ccgtggtggc cctgatctac 720

tgtaggaaga agcgcatctc cgccaactct acagatcccg tgaaggcagc acagttcgag 780

cctccaggca ggcagatgat cgccatccgg aagagacagc tggaggagac aaataacgat 840

tacgagaccg ccgacggcgg ctatatgaca ctgaatccac gcgcccccac cgacgatgac 900

aagaacatct atctgaccct gccccctaat gaccacgtga acagcaataa c 951

<210> 15
<211> 930
<212> DNA
<213> Artificial sequence
<220>
<223> Synthesized
<400> 15
atgggcatcc tgtccttcct gcccgtgctg gcaaccgagt ccgactgggc cgattgcaag 60

tctccacagc catggggaca catgctgctg tggaccgccg tgctgtttct ggcaccagtg 120

gcaggaacac ctgcagcacc acctaaggcc gtgctgaagc tggagcctca gtggatcaat 180

gtgctgcagg aggacagcgt gaccctgaca tgtcggggca cacactcccc agagtctgat 240

agcatccagt ggttccacaa tggcaacctg atcccaaccc acacacagcc ctcttaccgg 300

ttcaaggcca acaataacga cagcggcgag tatacctgcc agacaggcca gacctccctg 360

tctgatccag tgcacctgac agtgctgagc gagtggctgg tgctgcagac cccacacctg 420

gagttccagg agggcgagac aatcgtgctg aggtgtcact cctggaagga caagcccctg 480

gtgaaggtga ccttctttca gaatggcaag tccaagaagt tcagccgctc cgaccctaat 540

ttttctatcc cacaggccaa ccactctcac agcggcgatt accactgcac aggcaacatc 600

ggctacaccc tgtatagctc caagcctgtg accatcacag tgcaggcccc ttctagctcc 660

ccaatgggca tcatcgtggc agtggtgacc ggaatcgcag tggcagccat cgtggcagca 720

gtggtggccc tgatctactg ccggaagaag agaatctctg ccctgcctgg ctatccagag 780

tgtagggaga tgggcgagac actgcccgag aagcctgcca atccaaccaa cccagacgag 840

gcagataaag tgggagcaga gaataccatc acatatagcc tgctgatgca ccccgatgcc 900

ctggaggagc ctgacgatca gaacagaatc 930

<210>  16
<211>  472
<212>  PRT
<213>  Artificial sequence
<220>
<223>  Synthesized
<400>  16
Gln Val Gln Leu Gln Glu Ser Gly Pro Gly Leu Val Lys Pro Ser Glu
1               5                   10                  15

Thr Leu Ser Ile Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Leu Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  17
<211>  1416
<212>  DNA
<213>  Artificial sequence
<220>
<223>  Synthesized
<400>  17
caggtgcagc tgcaggagtc tggcccaggc ctggtgaagc cctctgagac cctgagcatc      60

acctgtacag tgagcggcag ctccctgaca tcctacggag tgcactgggt gcggcagcca     120

cctggcaagg gcctggaggg actgggcgtg atctggccag gaggcagcac caactataat     180

tccgccctga tgagccggct gacaatctct aaggacaaca gcaagtccca ggtgagcctg     240

aagatgtcta gcctgaccgc cgccgacaca gccatgtact attgcgcccg ggtgaccggc     300

acatggtact tcgacgtgtg gggccagggc accacagtga ccgtgtcctc tggcggcggc     360

ggctccggcg gcggcggatc cggaggagga ggcagcgaca tccagatgac ccagtcccca     420

agctccctgt ccgcctctct gggcgatagg gtgacaatca gctgctccgc ctctcagggc     480

atctccaact acctgaattg gtatcagcag aagccagacg gcaccgtgaa gctgctgatc     540

tactatacca gcacactgca ctccggagtg ccaagccggt tcagcggctc cggctctggc     600

accgacttta ccctgacaat ctctagcctg cagcccgagg atgtggccac atactattgt     660

cagcagtact ctaagctgcc ttggaccttc ggcggcggca caaagctgga gatcaaggat     720

aagcgggtgg agagcaagta tgggccccca tgcccaccat gcccagcacc tgagttcctt     780

gggggaccat cagtcttcct gttcccccca aaacccaagg acactctcat gatctcccgg     840

acccctgagg tcacgtgcgt ggtggtggac gtgagccagg aagaccccga ggtccagttc 900

aactggtacg tggatggcgt ggaggtgcat aatgccaaga caaagccgcg ggaggagcag 960

ttcaacagca cgtaccgtgt ggtcagcgtc ctcaccgtcc tgcaccagga ctggctgaac 1020

ggcaaggagt acaagtgcaa ggtctccaac aaaggcctcc cgtcctccat cgagaaaacc 1080

atctccaaag ccaaagggca gccccgagag ccacaggtgt acaccctgcc cccatcccag 1140

gaggagatga ccaagaacca ggtcagcctg acctgcctgg tcaaaggctt ctaccccagc 1200

gacatcgccg tggagtggga gagcaatggg cagccggaga acaactacaa gaccacgcct 1260

cccgtgctgg actccgacgg ctccttcttc ctctacagca gactaaccgt ggacaagagc 1320

aggtggcagg aggggaatgt cttctcatgc tccgtgatgc atgaggctct gcacaaccac 1380

tacacacaga agagcctctc cctgtctctg ggtaaa 1416

<210> 18
<211> 472
<212> PRT
<213> Artificial sequence
<220>
<223> Synthesized
<400> 18

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Val Thr Ile Ser Lys Asp Asn Ser Lys Ser Gln Val Ser Leu
65                  70                  75                  80

Lys Met Ser Ser Leu Thr Ala Ala Asp Thr Ala Val Tyr Tyr Cys Ala
                85                  90                  95

Arg Val Thr Gly Thr Trp Tyr Phe Asp Val Trp Gly Gln Gly Thr Thr

100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Val Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  19
<211>  472
<212>  PRT
<213>  Artificial sequence
<220>
<223>  Synthesized
<400>  19

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr

            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Val Thr Ile Ser Lys Asp Asn Ser Lys Ser Gln Val Ser Leu
65                  70                  75                  80

Lys Met Ser Ser Leu Thr Ala Ala Asp Thr Ala Val Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys

450                 455                 460

Ser Leu Ser Leu Ser Leu Gly Lys
465                 470

<210>  20
<211>  472
<212>  PRT
<213>  Artificial sequence
<220>
<223>  Synthesized
<400>  20

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Val Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Val Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr

370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  21
<211>  472
<212>  PRT
<213>  Artificial sequence
<220>
<223>  Synthesized
<400>  21

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Val Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val

290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  22
<211>  472
<212>  PRT
<213>  Artificial sequence
<220>
<223>  Synthesized
<400>  22

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Val Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Tyr Thr Leu Thr Ile Ser
        195                 200                 205

Ser Leu Gln Pro Glu Asp Ile Ala Thr Tyr Tyr Cys Gln Gln Tyr Ser

210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  23
<211>  472
<212>  PRT
<213>  Artificial sequence
<220>
<223>  Synthesized
<400>  23

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Leu Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser

130                 135                 140

Ala Ser Val Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  24
<211>  472
<212>  PRT
<213>  Artificial sequence
<220>
<223>  Synthesized
<400>  24

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met

50                  55                  60

Ser Arg Leu Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  25

<211>  472
<212>  PRT
<213>  Artificial sequence
<220>
<223>  Synthesized
<400>  25

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Leu Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Tyr Thr Leu Thr Ile Ser
        195                 200                 205

Ser Leu Gln Pro Glu Asp Ile Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr

                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210> 26
<211> 472
<212> PRT
<213> Artificial sequence
<220>
<223> Synthesized
<400> 26

Gln Val Gln Leu Gln Glu Ser Gly Pro Gly Leu Val Lys Pro Ser Glu
1               5                   10                  15

Thr Leu Ser Ile Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Leu Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Val Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Val Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln

                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210> 27
<211> 472
<212> PRT
<213> Artificial sequence
<220>
<223> Synthesized
<400> 27

Gln Val Gln Leu Gln Glu Ser Gly Pro Gly Leu Val Lys Pro Ser Glu
1               5                   10                  15

Thr Leu Ser Leu Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Val Thr Ile Ser Lys Asp Asn Ser Lys Ser Gln Val Ser Leu
65                  70                  75                  80

Lys Met Ser Ser Leu Thr Ala Ala Asp Thr Ala Val Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val
                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Tyr Thr Leu Thr Ile Ser
        195                 200                 205

Ser Leu Gln Pro Glu Asp Ile Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala

245                 250                 255

Pro Glu Phe Leu Gly Gly Pro Ser Val Phe Leu Phe Pro Pro Lys Pro
        260                 265                 270

Lys Asp Thr Leu Met Ile Ser Arg Thr Pro Glu Val Thr Cys Val Val
    275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

<210>  28
<211>  472
<212>  PRT
<213>  Artificial sequence
<220>
<223>  Synthesized
<400>  28

Gln Val Gln Leu Gln Glu Ser Gly Pro Gly Leu Val Lys Pro Ser Glu
1               5                   10                  15

Thr Leu Ser Ile Thr Cys Thr Val Ser Gly Ser Ser Leu Thr Ser Tyr
            20                  25                  30

Gly Val His Trp Val Arg Gln Pro Pro Gly Lys Gly Leu Glu Gly Leu
        35                  40                  45

Gly Val Ile Trp Pro Gly Gly Ser Thr Asn Tyr Asn Ser Ala Leu Met
    50                  55                  60

Ser Arg Leu Thr Ile Ser Lys Asp Asn Ser Lys Ser Gln Val Ser Leu
65                  70                  75                  80

Lys Met Ser Ser Leu Thr Ala Ala Asp Thr Ala Met Tyr Tyr Cys Ala
                85                  90                  95

Arg Val Thr Gly Thr Trp Tyr Phe Asp Val Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Leu Gly Asp Arg Val Thr Ile Ser Cys Ser Ala Ser Gln Gly
145                 150                 155                 160

Ile Ser Asn Tyr Leu Asn Trp Tyr Gln Gln Lys Pro Asp Gly Thr Val

                165                 170                 175

Lys Leu Leu Ile Tyr Tyr Thr Ser Thr Leu His Ser Gly Val Pro Ser
            180                 185                 190

Arg Phe Ser Gly Ser Gly Ser Gly Thr Asp Tyr Thr Leu Thr Ile Ser
        195                 200                 205

Ser Leu Gln Pro Glu Asp Ile Ala Thr Tyr Tyr Cys Gln Gln Tyr Ser
    210                 215                 220

Lys Leu Pro Trp Thr Phe Gly Gly Gly Thr Lys Leu Glu Ile Lys Asp
225                 230                 235                 240

Lys Arg Val Glu Ser Lys Tyr Gly Pro Pro Cys Pro Pro Cys Pro Ala
                245                 250                 255

Pro Glu Phe Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser Gln Glu Asp Pro Glu Val Gln Phe Asn Trp Tyr Val
    290                 295                 300

Asp Gly Val Glu Val His Asn Ala Lys Thr Lys Pro Arg Glu Glu Gln
305                 310                 315                 320

Phe Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Gln Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Arg Leu Thr Val Asp Lys Ser Arg Trp Gln Glu Gly Asn Val Phe
        435                 440                 445

Ser Cys Ser Val Met His Glu Ala Leu His Asn His Tyr Thr Gln Lys
    450                 455                 460

Ser Leu Ser Leu Ser Leu Gly Lys
465                 470

## Description

### Technical field

The present invention relates to the field of biotechnology, in particular to a molecule capable of binding to human 4-1BB and its application thereof.

### Background

4-1BB (CD137) is a member of the tumor necrosis factor (TNF) receptor family, which is encoded by the tumor necrosis factor receptor superfamily member 9 (TNFRSF9) gene. It was first discovered by Kwon and Weissman during the clonal differentiation screening process of activated murine T cells. Schwarzh et al. isolated human 4-1BB, which is homologous to murine 4-1BB, from human activated T cells and named it CD137. 4-1BB is a type I membrane glycoprotein. Different from PD-1/PD-L1, the expression of 4-1BB is activation-dependent. 4-1BB mediates the costimulatory signal for T cell activation, which is widely present on the surface of immune cells including activated NK cells, activated T cells, dendritic cells (DC), mast cells, monocytes, neutrophils and Treg cells.

Human 4-1BB has 255 amino acids and is expressed in the form of monomers and dimers on the cell surface, and is prone to form trimers with ligands, thereby turning on signal transduction.

Studies have shown that 4-1BB is an activated costimulatory molecule, and its signal pathway can promote the differentiation, proliferation, and cytokine production of T cells, especially CD8+ T cells. 4-1BB is mainly expressed on activated T cells and mainly acts at the middle and late stages of T cell immune responses. The 4-1BB signal pathway can achieve anti-tumor effects by enhancing the function of tumor-specific CD8+ T cells, and can also enhance the anti-tumor immune response mediated by CD8+ T cells by enhancing the immune function of NK cells, DC and CD4+ T cells. 4-1BB recruits TNFR-related factors TRAF1 and TRAF2 to form a heterotrimer, which promotes the production and secretion of cytokines through the c-Jun N-terminal kinase (JNK) pathway and the extracellular signal-regulated kinase (ERK) pathway, and through the β-catenin and AKT pathways enhancing signal transduction, and through the main transcription factor NF-κB regulating 4-1BB signal. In addition, the 4-1BB/4-1BBL interaction can improve the immune defense effect of T cell virus infection. Furthermore, 4-1BB monotherapy and combination therapy tumor models have confirmed durable anti-tumor protective T cell memory responses.

Currently, there is no agonist drug for 4-1BB on the market, and the fastest is in the clinical research and development stage, which cannot meet the needs of immunotherapy for various diseases including cancer. Developing an "accelerator" drug targeting 4-1BB to help activate the immune system using existing cancer drugs (such as Roche's Rituxan), develop new tumor immunotherapies, adapt to the market needs, and avoid the inferiority of drug resistance of PD-1/PD-L1, has a great application prospect.

### Invention Disclosure

The purpose of the present invention is to provide a molecule capable of binding to human 4-1BB and its application thereof.

The molecule provided by the present invention that can bind to human 4-1BB can be a humanized anti-human 4-1BB antibody or its antigen-binding fragments (such as Fab fragment, ScFv fragment) or an antibody fusion protein (such as antibody fusion protein formed by fusion of fully humanized antibody, ScFv, Fab fragment and other proteins) or an ADC antibody formed by anti-human 4-1BB humanized antibody conjugated with small molecule drugs with biological activity or a double antibody formed by fully humanized antibody, ScFv, Fab fragments and antibodies to other targets.

In the first aspect, the present invention claims an antibody.

The antibody claimed in the present invention, the amino acid sequences of HCDR1, HCDR2 and HCDR3 in the heavy chain variable region of the antibody are shown sequentially at positions 31-35, positions 50-64, and positions 98-106 in SEQ ID No. 1 from the N-terminus; the amino acid sequences of LCDR1, LCDR2 and LHCDR3 in the light chain variable region of the antibody are shown sequentially at positions 24-34, positions 50-56, and positions 89-97 in SEQ ID No. 2 from the N-terminus.

Furthermore, the amino acid sequence of the variable region of the heavy chain is any of the following:
(a1) Positions 1-117 in SEQ ID No.1 from the N-terminus (that is, positions 1-117 in SEQ ID No.16 from the N-terminus), or with more than 99%, more than 95%, more than 90%, more than 85%, more than 80% or more than 75% of consistency with positions 1-117 in SEQ ID No.1 from the N-terminus (that is, positions 1-117 in SEQ ID No.16 from the N-terminus) (the inconsistency is preferably in the framework region (FR));
(a2) Positions 1-117 in SEQ ID No.18 from the N-terminus, or with more than 99%, more than 95%, more than 90%, more than 85%, more than 80% or more than 75% of consistency with positions 1-117 in SEQ ID No.18 from the N-terminus (the inconsistency is preferably in the framework region (FR));
(a3) Positions 1-117 in SEQ ID No.19 from the N-terminus, or with more than 99%, more than 95%, more than 90%, more than 85%, more than 80% or more than 75% of consistency with positions 1-117 in SEQ ID No.19 from the N-terminus (the inconsistency is preferably in the framework region (FR));
(a4) Positions 1-117 in SEQ ID No.20 from the N-terminus, or with more than 99%, more than 95%, more than 90%, more than 85%, more than 80% or more than 75% of consistency with positions 1-117 in SEQ ID No.20 from the N-terminus (the inconsistency is preferably in the framework region (FR));
(a5) Positions 1-117 in SEQ ID No.21 from the N-terminus, or with more than 99%, more than 95%, more than 90%, more than 85%, more than 80% or more than 75% of consistency with positions 1-117 in SEQ ID No.21 from the N-terminus (the inconsistency is preferably in the framework region (FR));
(a6) Positions 1-117 in SEQ ID No.22 from the N-terminus, or with more than 99%, more than 95%, more than 90%, more than 85%, more than 80% or more than 75% of consistency with positions 1-117 in SEQ ID No.22 from the N-terminus (the inconsistency is preferably in the framework region (FR));
(a7) Positions 1-117 in SEQ ID No.23 from the N-terminus, or with more than 99%, more than 95%, more than 90%, more than 85%, more than 80% or more than 75% of consistency with positions 1-117 in SEQ ID No.23 from the N-terminus (the inconsistency is preferably in the framework region (FR));
(a8) Positions 1-117 in SEQ ID No.24 from the N-terminus, or with more than 99%, more than 95%, more than 90%, more than 85%, more than 80% or more than 75% of consistency with positions 1-117 in SEQ ID No.24 from the N-terminus (the inconsistency is preferably in the framework region (FR));
(a9) Positions 1-117 in SEQ ID No.25 from the N-terminus, or with more than 99%, more than 95%, more than 90%, more than 85%, more than 80% or more than 75% of consistency with positions 1-117 in SEQ ID No.25 from the N-terminus (the inconsistency is preferably in the framework region (FR));
(a10) Positions 1-117 in SEQ ID No.26 from the N-terminus, or with more than 99%, more than 95%, more than 90%, more than 85%, more than 80% or more than 75% of consistency with positions 1-117 in SEQ ID No.26 from the N-terminus (the inconsistency is preferably in the framework region (FR));
(all) Positions 1-117 in SEQ ID No.27 from the N-terminus, or with more than 99%, more than 95%, more than 90%, more than 85%, more than 80% or more than 75% of consistency with positions 1-117 in SEQ ID No.27 from the N-terminus (the inconsistency is preferably in the framework region (FR));
(a12) Positions 1-117 in SEQ ID No.28 from the N-terminus, or with more than 99%, more than 95%, more than 90%, more than 85%, more than 80% or more than 75% of consistency with positions 1-117 in SEQ ID No.28 from the N-terminus (the inconsistency is preferably in the framework region (FR)).

Furthermore, the amino acid sequence of the variable region of the light chain is any of the following:
(b1) Positions 1-107 in SEQ ID No.2 from the N-terminus (that is, positions 133-239 in SEQ ID No.16 from the N-terminus), or with more than 99%, more than 95%, more than 90%, more than 85%, more than 80% or more than 75% of consistency with positions 1-107 in SEQ ID No.2 from the N-terminus (that is, positions 133-239 in SEQ ID No.16 from the N-terminus) (the inconsistency is preferably in the framework region (FR));
(b2) Positions 133-239 in SEQ ID No.18 from the N-terminus, or with more than 99%, more than 95%, more than 90%, more than 85%, more than 80% or more than 75% of consistency with positions 133-239 in SEQ ID No.18 from the N-terminus (the inconsistency is preferably in the framework region (FR));
(b3) Positions 133-239 in SEQ ID No.19 from the N-terminus, or with more than 99%, more than 95%, more than 90%, more than 85%, more than 80% or more than 75% of consistency with positions 133-239 in SEQ ID No.19 from the N-terminus (the inconsistency is preferably in the framework region (FR));
(b4) Positions 133-239 in SEQ ID No.20 from the N-terminus, or with more than 99%, more than 95%, more than 90%, more than 85%, more than 80% or more than 75% of consistency with positions 133-239 in SEQ ID No.20 from the N-terminus (the inconsistency is preferably in the framework region (FR));
(b5) Positions 133-239 in SEQ ID No.21 from the N-terminus, or with more than 99%, more than 95%, more than 90%, more than 85%, more than 80% or more than 75% of consistency with positions 133-239 in SEQ ID No.21 from the N-terminus (the inconsistency is preferably in the framework region (FR));
(b6) Positions 133-239 in SEQ ID No.22 from the N-terminus, or with more than 99%, more than 95%, more than 90%, more than 85%, more than 80% or more than 75% of consistency with positions 133-239 in SEQ ID No.22 from the N-terminus (the inconsistency is preferably in the framework region (FR));
(b7) Positions 133-239 in SEQ ID No.23 from the N-terminus, or with more than 99%, more than 95%, more than 90%, more than 85%, more than 80% or more than 75% of consistency with positions 133-239 in SEQ ID No.23 from the N-terminus (the inconsistency is preferably in the framework region (FR));
(b8) Positions 133-239 in SEQ ID No.24 from the N-terminus, or with more than 99%, more than 95%, more than 90%, more than 85%, more than 80% or more than 75% of consistency with positions 133-239 in SEQ ID No.24 from the N-terminus (the inconsistency is preferably in the framework region (FR));
(b9) Positions 133-239 in SEQ ID No.25 from the N-terminus, or with more than 99%, more than 95%, more than 90%, more than 85%, more than 80% or more than 75% of consistency with positions 133-239 in SEQ ID No.25 from the N-terminus (the inconsistency is preferably in the framework region (FR));
(b10) Positions 133-239 in SEQ ID No.26 from the N-terminus, or with more than 99%, more than 95%, more than 90%, more than 85%, more than 80% or more than 75% of consistency with positions 133-239 in SEQ ID No.26 from the N-terminus (the inconsistency is preferably in the framework region (FR));
(b11) Positions 133-239 in SEQ ID No.27 from the N-terminus, or with more than 99%, more than 95%, more than 90%, more than 85%, more than 80% or more than 75% of consistency with positions 133-239 in SEQ ID No.27 from the N-terminus (the inconsistency is preferably in the framework region (FR));
(b12) Positions 133-239 in SEQ ID No.28 from the N-terminus, or with more than 99%, more than 95%, more than 90%, more than 85%, more than 80% or more than 75% of consistency with positions 133-239 in SEQ ID No.28 from the N-terminus (the inconsistency is preferably in the framework region (FR)).

The heavy chain constant region of the antibody is a human IgG4 constant region, and the specific amino acid sequence is shown at positions 118-444 in SEQ ID No.1 from the N-terminus. The light chain constant region of the antibody is a constant region of human light chain kappa, and the specific amino acid sequence is shown at positions 108-214 in SEQ ID No.2 from the N-terminus.

Furthermore, the amino acid sequence of the heavy chain is SEQ ID No.1, or with more than 99%, more than 95%, more than 90%, and more than 85%, more than 80% or more than 75% of consistency with SEQ ID No.1 (the inconsistency is preferably in the framework region (FR)). The amino acid sequence of the light chain is SEQ ID No.2, or with more than 99%, more than 95%, more than 90%, and more than 85%, more than 80% or more than 75% of consistency with SEQ ID No.2 (the inconsistency is preferably in the framework region (FR)).

The antibody of the present invention can be of any type, such as IgG, IgM, IgE, IgA or IgD. Preferably, the 4-1BB antibody is the IgG type, such as IgG1, IgG2, IgG3 or IgG4 subtype, more preferably IgG4 subtype.

The light chain type of the antibody of the present invention can be either a kappa chain or a lambda chain, preferably a Kappa chain.

In a specific embodiment of the present invention, the antibody is specifically a humanized anti-human 4-1BB antibody Hanke10F4. The Hanke10F4 mainly binds to Domain 1 and Domain 2A of the extracellular domain of human 4-1BB.

The antibodies provided by the present invention have one or more of the following properties or characteristics:
1) Specific binding to human 4-1BB;
2) Binding to human and cynomolgus monkey 4-1BB;
3) Binding to human or cynomolgus monkey 4-1BB;
4) To a certain extent, it can block the binding of human 4-1BBL (4-1BB ligand) to human 4-1BB;
5) It is IgG, such as IgG1, IgG2, IgG3 or IgG4;
6) It is a human antibody, or a humanized antibody.

The antibodies or antigen-binding fragments thereof of the present invention have agonist activity to human 4-1BB, and can stimulate the proliferation of CD4+T and CD8+T cells. They can activate the downstream NF- κ B signaling pathway by binding to 4-1BB molecules on the cell surface, thereby significantly promoting the production and secretion of cytokine IFN- γ and significantly inhibiting the occurrence and development of tumors in humanized mice with human 4-1BB gene knock-in. To a certain extent, it indicates that the antibodies or their antigen-binding parts can regulate the immune system by regulating the activity of immune cells, and can be used as immune enhancers for anti-tumor or antiviral immune responses of immune enhancers, or immune modulators for T cell-mediated autoimmune diseases.

The antibodies of the present invention can be prepared by methods known in the art, and can be prepared by expression in transfected cells such as immortalized eukaryotic cells such as myeloma or hybridoma cells. The antibodies can be converted from one type or subtype to another type or subtype by methods known in the art. In addition, the antibodies provided by the present invention can be a monoclonal antibody or a polyclonal antibody, preferably a monoclonal antibody.

The antibodies of the present invention can be produced by techniques known in the art, including conventional monoclonal antibody techniques, such as standard somatic cell hybridization techniques, virus or oncogene transformation of B lymphocytes, or recombinant antibody technology, as described in detail below.

Hybridoma production is a commonly used method and is known in the art. First, human 4-1BB protein is transiently transfected and expressed to immunize Balb/c mice. The amino acid sequence of the human 4-1BB antigen is shown in SEQ ID No. 5 (positions 24-184 are the extracellular region). The immune antigen is isolated or purified human 4-1BB, which may be a fragment of human 4-1BB, such as the extracellular domain of human 4-1BB. Animal immunization can be carried out by any method known in the art, immunizing non-human animals such as mice, rats, sheep, and the like. After immunizing the animal with the human 4-1BB antigen, an antibody-producing immortalized cell line is prepared from the cells isolated from the immunized animal. After immunization, the animals are sacrificed to obtain immortalized lymph nodes and/or splenic B cells, and cell fusion is carried out by conventional methods, and positive cell clones are screened by ELISA, which are cells that produce anti-4-1BB antibodies. Then subcloning is repeated for further screening to select hybridomas with good growth status, high antibody production, and 100% positive culture supernatant. Hybridomas can be expanded in syngeneic animals, animals lacking an immune system, and nude mice, or expanded in cell culture in vitro. Methods of selection, cloning and expansion of hybridomas are well known to those skilled in the art. The subtype of immunoglobulin secreted by hybridoma cells was detected as IgG1 by the biphasic agar diffusion test. The antibodies of the present invention can also be prepared by phage display methods. This phage display method for isolating human antibodies has been established in the art.

After the antibody is produced and expressed, it contains antibody, dimer, single light and heavy chains, or other immunoglobulin forms, which can be purified according to standard methods in the art, such as ammonium sulfate precipitation, affinity column, column chromatography, gel electrophoresis, etc.

In the second aspect, the present invention claims the antigen-binding fragments of the aforementioned antibodies.

Wherein, the antigen-binding fragments may include one or more of the following: (1) the light chain of the aforementioned antibody; (2) the heavy chain of the aforementioned antibody; (3) the variable region of the light chain of the aforementioned antibody; (4) the variable region of the heavy chain of the aforementioned antibody; (5) one or more CDR regions of the aforementioned antibody.

Furthermore, the antigen-binding fragments may be any one of the following: (1) Fab fragment, which is a monovalent fragment composed of VL, VH, CL and CH1 domains; (2) F(ab')2 fragment, which is a bivalent fragment, including two Fab fragments of which the hinge region is linked by disulfide bonds; (3) Fd fragment, composed of VH and CH1 domains; (4) FV fragment, composed of the VL and VH domains of one arm of the antibody; (5) Isolated CDR (eg CDR from the light chain and/or CDR from the heavy chain); (6) Single-chain antibody, etc., among which the single-chain antibody can have a scFv structure, and the single-chain antibody can carry different tags, such as scFv-His, scFv-Fc, etc..

In a specific embodiment of the present invention, the antigen-binding fragment provided by the present invention is a single-chain antibody with a tag (scFv-Fc). The single-chain antibody (scFv-Fc) may consist of a human IgG4 Fc tag comprising a single polypeptide chain linked to the VL domain of the VH domain, wherein the VL domain and VH domain are paired to form a monovalent molecule. Single-chain antibodies can be prepared according to methods known in the art.

In this aspect, the present invention specifically claims an anti-4-1BB single-chain antibody.

The anti-4-1BB single-chain antibody claimed in the present invention is formed by connecting a heavy chain variable region and a light chain variable region; the amino acid sequences of HCDR1, HCDR2 and HCDR3 in the variable region of the heavy chain are shown sequentially at positions 31-35, positions 50-64, and positions 98-106 in SEQ ID No. 16 from the N-terminus; the amino acid sequences of LCDR1, LCDR2 and LHCDR3 in the variable region of the light chain are shown sequentially at positions 156-166, positions 182-188, and positions 221-229 in SEQ ID No. 16 from the N-terminus.

Furthermore, the amino acid sequence of the heavy chain variable region of the single-chain antibody is any one of the following:
(a1) Positions 1-117 in SEQ ID No.16 from the N-terminus, or with more than 99%, more than 95%, more than 90%, more than 85%, more than 80% or more than 75% of consistency with positions 1-117 in SEQ ID No.16 from the N-terminus(the inconsistency is preferably in the framework region (FR));
(a2) Positions 1-117 in SEQ ID No.18 from the N-terminus, or with more than 99%, more than 95%, more than 90%, more than 85%, more than 80% or more than 75% of consistency with positions 1-117 in SEQ ID No.18 from the N-terminus (the inconsistency is preferably in the framework region (FR));
(a3) Positions 1-117 in SEQ ID No.19 from the N-terminus, or with more than 99%, more than 95%, more than 90%, more than 85%, more than 80% or more than 75% of consistency with positions 1-117 in SEQ ID No.19 from the N-terminus (the inconsistency is preferably in the framework region (FR));
(a4) Positions 1-117 in SEQ ID No.20 from the N-terminus, or with more than 99%, more than 95%, more than 90%, more than 85%, more than 80% or more than 75% of consistency with positions 1-117 in SEQ ID No.20 from the N-terminus (the inconsistency is preferably in the framework region (FR));
(a5) Positions 1-117 in SEQ ID No.21 from the N-terminus, or with more than 99%, more than 95%, more than 90%, more than 85%, more than 80% or more than 75% of consistency with positions 1-117 in SEQ ID No.21 from the N-terminus (the inconsistency is preferably in the framework region (FR));
(a6) Positions 1-117 in SEQ ID No.22 from the N-terminus, or with more than 99%, more than 95%, more than 90%, more than 85%, more than 80% or more than 75% of consistency with positions 1-117 in SEQ ID No.22 from the N-terminus (the inconsistency is preferably in the framework region (FR));
(a7) Positions 1-117 in SEQ ID No.23 from the N-terminus, or with more than 99%, more than 95%, more than 90%, more than 85%, more than 80% or more than 75% of consistency with positions 1-117 in SEQ ID No.23 from the N-terminus (the inconsistency is preferably in the framework region (FR));
(a8) Positions 1-117 in SEQ ID No.24 from the N-terminus, or with more than 99%, more than 95%, more than 90%, more than 85%, more than 80% or more than 75% of consistency with positions 1-117 in SEQ ID No.24 from the N-terminus (the inconsistency is preferably in the framework region (FR));
(a9) Positions 1-117 in SEQ ID No.25 from the N-terminus, or with more than 99%, more than 95%, more than 90%, more than 85%, more than 80% or more than 75% of consistency with positions 1-117 in SEQ ID No.25 from the N-terminus (the inconsistency is preferably in the framework region (FR));
(a10) Positions 1-117 in SEQ ID No.26 from the N-terminus, or with more than 99%, more than 95%, more than 90%, more than 85%, more than 80% or more than 75% of consistency with positions 1-117 in SEQ ID No.26 from the N-terminus (the inconsistency is preferably in the framework region (FR));
(all) Positions 1-117 in SEQ ID No.27 from the N-terminus, or with more than 99%, more than 95%, more than 90%, more than 85%, more than 80% or more than 75% of consistency with positions 1-117 in SEQ ID No.27 from the N-terminus (the inconsistency is preferably in the framework region (FR));
(a12) Positions 1-117 in SEQ ID No.28 from the N-terminus, or with more than 99%, more than 95%, more than 90%, more than 85%, more than 80% or more than 75% of consistency with positions 1-117 in SEQ ID No.28 from the N-terminus (the inconsistency is preferably in the framework region (FR)).

Furthermore, the amino acid sequence of the light chain variable region of the single-chain antibody can be any of the following:
(b1) Positions 133-239 in SEQ ID No.16 from the N-terminus, or with more than 99%, more than 95%, more than 90%, more than 85%, more than 80% or more than 75% of consistency with positions 133-239 in SEQ ID No.16 from the N-terminus (the inconsistency is preferably in the framework region (FR));
(b2) Positions 133-239 in SEQ ID No.18 from the N-terminus, or with more than 99%, more than 95%, more than 90%, more than 85%, more than 80% or more than 75% of consistency with positions 133-239 in SEQ ID No.18 from the N-terminus (the inconsistency is preferably in the framework region (FR));
(b3) Positions 133-239 in SEQ ID No.19 from the N-terminus, or with more than 99%, more than 95%, more than 90%, more than 85%, more than 80% or more than 75% of consistency with positions 133-239 in SEQ ID No.19 from the N-terminus (the inconsistency is preferably in the framework region (FR));
(b4) Positions 133-239 in SEQ ID No.20 from the N-terminus, or with more than 99%, more than 95%, more than 90%, more than 85%, more than 80% or more than 75% of consistency with positions 133-239 in SEQ ID No.20 from the N-terminus (the inconsistency is preferably in the framework region (FR));
(b5) Positions 133-239 in SEQ ID No.21 from the N-terminus, or with more than 99%, more than 95%, more than 90%, more than 85%, more than 80% or more than 75% of consistency with positions 133-239 in SEQ ID No.21 from the N-terminus (the inconsistency is preferably in the framework region (FR));
(b6) Positions 133-239 in SEQ ID No.22 from the N-terminus, or with more than 99%, more than 95%, more than 90%, more than 85%, more than 80% or more than 75% of consistency with positions 133-239 in SEQ ID No.22 from the N-terminus (the inconsistency is preferably in the framework region (FR));
(b7) Positions 133-239 in SEQ ID No.23 from the N-terminus, or with more than 99%, more than 95%, more than 90%, more than 85%, more than 80% or more than 75% of consistency with positions 133-239 in SEQ ID No.23 from the N-terminus (the inconsistency is preferably in the framework region (FR));
(b8) Positions 133-239 in SEQ ID No.24 from the N-terminus, or with more than 99%, more than 95%, more than 90%, more than 85%, more than 80% or more than 75% of consistency with positions 133-239 in SEQ ID No.24 from the N-terminus (the inconsistency is preferably in the framework region (FR));
(b9) Positions 133-239 in SEQ ID No.25 from the N-terminus, or with more than 99%, more than 95%, more than 90%, more than 85%, more than 80% or more than 75% of consistency with positions 133-239 in SEQ ID No.25 from the N-terminus (the inconsistency is preferably in the framework region (FR));
(b10) Positions 133-239 in SEQ ID No.26 from the N-terminus, or with more than 99%, more than 95%, more than 90%, more than 85%, more than 80% or more than 75% of consistency with positions 133-239 in SEQ ID No.26 from the N-terminus (the inconsistency is preferably in the framework region (FR));
(b11) Positions 133-239 in SEQ ID No.27 from the N-terminus, or with more than 99%, more than 95%, more than 90%, more than 85%, more than 80% or more than 75% of consistency with positions 133-239 in SEQ ID No.27 from the N-terminus (the inconsistency is preferably in the framework region (FR));
(b12) Positions 133-239 in SEQ ID No.28 from the N-terminus, or with more than 99%, more than 95%, more than 90%, more than 85%, more than 80% or more than 75% of consistency with positions 133-239 in SEQ ID No.28 from the N-terminus (the inconsistency is preferably in the framework region (FR)).

Furthermore, the amino acid sequence of the single-chain antibody can be any of the following:
(c1) Positions 1-239 of SEQ ID No.16, or with more than 99%, more than 95%, more than 90%, more than 85%, more than 80% or more than 75% of consistency with positions 1-239 of SEQ ID No.16 (the inconsistency is preferably in the framework region (FR));
(c2) Positions 1-239 of SEQ ID No.18, or with more than 99%, more than 95%, more than 90%, more than 85%, more than 80% or more than 75% of consistency with positions 1-239 of SEQ ID No.18 (the inconsistency is preferably in the framework region (FR));
(c3) Positions 1-239 of SEQ ID No.19, or with more than 99%, more than 95%, more than 90%, more than 85%, more than 80% or more than 75% of consistency with positions 1-239 of SEQ ID No.19 (the inconsistency is preferably in the framework region (FR));
(c4) Positions 1-239 of SEQ ID No.20, or with more than 99%, more than 95%, more than 90%, more than 85%, more than 80% or more than 75% of consistency with positions 1-239 of SEQ ID No.20 (the inconsistency is preferably in the framework region (FR)) (the inconsistency is preferably in the framework region (FR));
(c5) Positions 1-239 of SEQ ID No.21, or with more than 99%, more than 95%, more than 90%, more than 85%, more than 80% or more than 75% of consistency with positions 1-239 of SEQ ID No.21 (the inconsistency is preferably in the framework region (FR));
(c6) Positions 1-239 of SEQ ID No.22, or with more than 99%, more than 95%, more than 90%, more than 85%, more than 80% or more than 75% of consistency with positions 1-239 of SEQ ID No.22 (the inconsistency is preferably in the framework region (FR));
(c7) Positions 1-239 of SEQ ID No.23, or with more than 99%, more than 95%, more than 90%, more than 85%, more than 80% or more than 75% of consistency with positions 1-239 of SEQ ID No.23 (the inconsistency is preferably in the framework region (FR));
(c8) Positions 1-239 of SEQ ID No.24, or with more than 99%, more than 95%, more than 90%, more than 85%, more than 80% or more than 75% of consistency with positions 1-239 of SEQ ID No.24 (the inconsistency is preferably in the framework region (FR));
(c9) Positions 1-239 of SEQ ID No.25, or with more than 99%, more than 95%, more than 90%, more than 85%, more than 80% or more than 75% of consistency with positions 1-239 of SEQ ID No.25 (the inconsistency is preferably in the framework region (FR));
(c10) Positions 1-239 of SEQ ID No.26, or with more than 99%, more than 95%, more than 90%, more than 85%, more than 80% or more than 75% of consistency with positions 1-239 of SEQ ID No.26 (the inconsistency is preferably in the framework region (FR));
(c11) Positions 1-239 of SEQ ID No.27, or with more than 99%, more than 95%, more than 90%, more than 85%, more than 80% or more than 75% of consistency with positions 1-239 of SEQ ID No.27 (the inconsistency is preferably in the framework region (FR));
(c12) Positions 1-239 of SEQ ID No.28, or with more than 99%, more than 95%, more than 90%, more than 85%, more than 80% or more than 75% of consistency with positions 1-239 of SEQ ID No.28 (the inconsistency is preferably in the framework region (FR)).

In the third aspect, the present invention claims an antibody fusion protein formed by the aforementioned antibodies or the aforementioned antigen-binding fragments and other proteins.

Wherein, the other proteins may be toxin, enzyme, cell membrane protein molecule, cytokine or receptor protein, etc.

Furthermore, the antibody fusion protein claimed in the present invention may be a single-chain antibody with a tag protein formed by the aforementioned single-chain antibody and a tag protein.

In a specific embodiment of the present invention, the tag protein is Fc.

Furthermore, the Fc may be derived from, for example, IgG, IgM, IgE, IgA or IgD. Preferably, the Fc is from the IgG type, such as IgG1, IgG2, IgG3 or IgG4 subtype, more preferably IgG4 subtype. The Fc is preferably of human origin.

Furthermore, the amino acid sequence of the Fc is shown at positions 240-472 of SEQ ID No.16, or with more than 99%, more than 95%, more than 90%, and more than 85%, more than 80% or more than 75% of consistency with positions 240-472 in SEQ ID No.16 from the N-terminus.

More specifically, the amino acid sequence of the single-chain antibody with the tagged protein may be any of the following:
(d1) As shown in SEQ ID No.16, or with more than 99%, more than 95%, more than 90%, more than 85%, more than 80% or more than 75% of consistency with SEQ ID No.16 (the inconsistency is preferably in the framework region (FR));
(d2) As shown in SEQ ID No.18, or with more than 99%, more than 95%, more than 90%, more than 85%, more than 80% or more than 75% of consistency with SEQ ID No.18 (the inconsistency is preferably in the framework region (FR));
(d3) As shown in SEQ ID No.19, or with more than 99%, more than 95%, more than 90%, more than 85%, more than 80% or more than 75% of consistency with SEQ ID No.19 (the inconsistency is preferably in the framework region (FR));
(d4) As shown in SEQ ID No.20, or with more than 99%, more than 95%, more than 90%, more than 85%, more than 80% or more than 75% of consistency with SEQ ID No.20 (the inconsistency is preferably in the framework region (FR));
(d5) As shown in SEQ ID No.21, or with more than 99%, more than 95%, more than 90%, more than 85%, more than 80% or more than 75% of consistency with SEQ ID No.21 (the inconsistency is preferably in the framework region (FR));
(d6) As shown in SEQ ID No.22, or with more than 99%, more than 95%, more than 90%, more than 85%, more than 80% or more than 75% of consistency with SEQ ID No.22 (the inconsistency is preferably in the framework region (FR));
(d7) As shown in SEQ ID No.23, or with more than 99%, more than 95%, more than 90%, more than 85%, more than 80% or more than 75% of consistency with SEQ ID No.23 (the inconsistency is preferably in the framework region (FR));
(d8) As shown in SEQ ID No.24, or with more than 99%, more than 95%, more than 90%, more than 85%, more than 80% or more than 75% of consistency with SEQ ID No.24 (the inconsistency is preferably in the framework region (FR));
(d9) As shown in SEQ ID No.25, or with more than 99%, more than 95%, more than 90%, more than 85%, more than 80% or more than 75% of consistency with SEQ ID No.25 (the inconsistency is preferably in the framework region (FR));
(d10) As shown in SEQ ID No.26, or with more than 99%, more than 95%, more than 90%, more than 85%, more than 80% or more than 75% of consistency with SEQ ID No.26 (the inconsistency is preferably in the framework region (FR));
(d11) As shown in SEQ ID No.27, or with more than 99%, more than 95%, more than 90%, more than 85%, more than 80% or more than 75% of consistency with SEQ ID No.27 (the inconsistency is preferably in the framework region (FR));
(d12) As shown in SEQ ID No.28, or with more than 99%, more than 95%, more than 90%, more than 85%, more than 80% or more than 75% of consistency with SEQ ID No.28 (the inconsistency is preferably in the framework region (FR)).

In the fourth aspect, the present invention claims a double antibody formed by the aforementioned antibody or the aforementioned antigen-binding fragment or the aforementioned single-chain antibody with a tag protein and antibody of other targets.

In the fifth aspect, the present invention claims a nucleic acid molecule.

The nucleic acid molecule claimed in the present invention encodes the aforementioned antibody or the aforementioned antigen-binding fragment or the aforementioned antibody fusion protein or the aforementioned double antibody.

The nucleic acid molecule of the present invention may be DNA or RNA, and may or may not contain intronic sequences. Preferably, the nucleic acid molecule is a cDNA molecule.

Furthermore, in the nucleic acid molecule, the nucleotide sequences encoding HCDR1, HCDR2 and HCDR3 in the heavy chain variable region of the antibody are at positions 91-105, positions 148-192, and positions 292-318 sequentially in SEQ ID No.3 from the 5'end; the nucleotide sequences encoding LCDR1, LCDR2 and LHCDR3 in the light chain variable region of the antibody are at positions 70-102, positions 148-168, and positions 265-291 sequentially in SEQ ID No.4 from the 5'end.

Furthermore, in the nucleic acid molecule, the nucleotide sequence encoding the heavy chain variable region of the antibody is at positions 1-351 in SEQ ID No.3 from the 5'end, or with more than 99%, more than 95%, more than 90%, more than 85%, more than 80% or more than 75% of consistency with positions 1-351 in SEQ ID No.3 from the 5'end (the inconsistency is preferably in the framework region (FR)). The nucleotide sequence encoding the light chain variable region of the antibody is at positions 1-321 in SEQ ID No.4 from the 5'end, or with more than 99%, more than 95%, more than 90%, more than 85%, more than 80% or more than 75% of consistency with positions 1-321 in SEQ ID No.4 from the 5'end (the inconsistency is preferably in the framework region (FR)).

In the nucleic acid molecule, the nucleotide sequence encoding the variable region of the heavy chain constant region of the antibody is at positions 352-1332 in SEQ ID No.3 from the 5'end. The nucleotide sequence of the constant variable region of the light chain encoding the antibody is at positions 322-642 in SEQ ID No.4 from the 5'end.

More specifically, in the nucleic acid molecule, the nucleotide sequence encoding the heavy chain variable region of the antibody is SEQ ID No.3, or with more than 99%, more than 95%, more than 90%, more than 85%, more than 80% or more than 75% of consistency with SEQ ID No.3 (the inconsistency is preferably in the framework region (FR)). The nucleotide sequence encoding the light chain variable region of the antibody is SEQ ID No.4, or with more than 99%, more than 95%, more than 90%, more than 85%, more than 80% or more than 75% of consistency with SEQ ID No.4 (the inconsistency is preferably in the framework region (FR)).

The nucleic acid molecules of the present invention can be obtained using appropriate molecular biology techniques. For the antibody expressed by the hybridoma, cDNA encoding the light chain and the heavy chain of the antibody prepared by the hybridoma can be obtained by PCR amplification or cDNA cloning technology.

The nucleic acid molecule of the present invention can encode the amino acid sequence of a part of a light chain or a heavy chain, a full-length light chain or heavy chain, or an antibody derivative or an antigen-binding fragment thereof.

The isolated DNA encoding VH can be converted into a full-length heavy chain gene by ligating the DNA encoding VH to another DNA molecule encoding the constant region (CH1, CH2 and CH3) of the heavy chain. Human heavy chain constant region gene sequences are known in the art, and DNA fragments containing these regions can be obtained by standard PCR amplification. The heavy chain constant region can be an IgG1, IgG2, IgG3, IgG4, IgA, IgE, IgM, or IgD constant region. Preferred is the IgG4 constant region.

The isolated DNA encoding VL region can be converted into a full-length light chain gene by ligating the DNA encoding VL to another DNA molecule encoding the constant region CL of the light chain. Human light chain constant region gene sequences are known in the art, and DNA fragments containing these regions can be obtained by standard PCR amplification. The light chain constant region can be a Kappa or Lambda constant region.

In this aspect, the present invention specifically claims nucleic acid molecules encoding the aforementioned single-chain antibody or the aforementioned single-chain antibody with a tag protein or the aforementioned fusion antibody.

Furthermore, in the nucleic acid molecule, the nucleotide sequences encoding HCDR1, HCDR2 and HCDR3 in the heavy chain variable region are at positions 91-105, positions 148-192, and positions 292-318 sequentially in SEQ ID No.17 from the 5'end; the nucleotide sequences encoding LCDR1, LCDR2 and LHCDR3 in the light chain variable region are at positions 466-498, positions 544-564, and positions 661-687 sequentially in SEQ ID No.17 from the 5'end.

Furthermore, in the nucleic acid molecule, the nucleotide sequence encoding the heavy chain variable region is at positions 1-351 in SEQ ID No.17 from the 5'end, or with more than 99%, more than 95%, more than 90%, more than 85%, more than 80% or more than 75% of consistency with positions 1-351 in SEQ ID No.17 from the 5'end (the inconsistency is preferably in the framework region (FR)). The nucleotide sequence encoding the light chain variable region is at positions 397-717 in SEQ ID No.17 from the 5'end, or with more than 99%, more than 95%, more than 90%, more than 85%, more than 80% or more than 75% of consistency with positions 397-717 in SEQ ID No.17 from the 5'end (the inconsistency is preferably in the framework region (FR)).

More specifically, the nucleotide sequence encoding the single-chain antibody is at positions 1-717 in SEQ ID No.17 from the 5'end, or with more than 99%, more than 95%, more than 90%, more than 85%, more than 80% or more than 75% of consistency with positions 1-717 in SEQ ID No.17 from the 5'end (the inconsistency is preferably in the framework region (FR)).

More specifically, the nucleotide sequence encoding the single-chain antibody with the tagged protein is SEQ ID No.17, or with more than 99%, more than 95%, more than 90%, more than 85%, more than 80% or more than 75% of consistency with SEQ ID No.17 (the inconsistency is preferably in the framework region (FR)).

The nucleic acid molecules of the present invention can be obtained using appropriate molecular biology techniques.

In the sixth aspect, the present invention claims the expression cassettes, recombinant vectors, and recombinant cells containing the aforementioned nucleic acid molecules.

Wherein, the carrier can be used for binding molecules such as antibody or antigen-binding fragment thereof (such as single-chain antibody) or a single-chain antibody with a tagged protein (such as a single-chain antibody fused with Fc) or an expression vector for expression of the fusion antibody.

In order to express the antibody (or single-chain antibody or fusion antibody) of the present invention, DNA encoding partial or full-length light chain and heavy chain is inserted into an expression vector to start the transcription and translation of the DNA molecule. The antibody light chain gene and the antibody heavy chain gene can be inserted into a separate vector or inserted into the same expression vector. The insertion method can be any suitable method known, such as linking the complementary restriction sites of the antibody gene fragment to the vector. The light chain and heavy chain variable regions of the antibodies described in the present invention can be used to generate full-length antibody genes of any antibody type and subtype.

The present invention further provides a host cell (ie, the recombinant cell) containing the nucleic acid molecule provided by the present invention. The host cell may be any cell available for expression vectors. For example, higher eukaryotic host cells, such as mammalian cells including, for example, Chinese Hamster Ovary (CHO) cells; lower eukaryotic host cells, such as yeast cells, and prokaryotic cells, such as bacterial cells, Escherichia coli, etc. Transfection methods for introducing recombinant nucleic acid constructs into host cells include electroporation, calcium phosphate transfection, DEAE-dextran, lipofection, and phage infection, etc. The expression vector encoding the antibody gene can be introduced into the host cell by known methods, and the host cell is cultured enough to allow the binding molecule to be expressed in the host cell to produce antibody.

The vector can be a plasmid, cosmid, phage or virus vector. The plasmid can be pCDNA3.4 and pCDNA3.4-L or others.

Those of ordinary skill in the art can easily use known methods, such as directed evolution and point mutation methods, to mutate the nucleotide sequence of the anti-4-1BB antibody (or single-chain antibody or fusion antibody) of the present invention. Those nucleotides with more than 99%, more than 95%, more than 90%, more than 85%, more than 80% or more than 75% of consistency with the nucleotide sequence of the anti-4-1BB antibody (or single-chain antibody or fusion antibody) of the present invention, which have been artificially modified, as long as they encode the anti-4-1BB antibody and have the anti-4-1BB antibody activity, they are all derived from the nucleotide sequence of the present invention and are equivalent to the sequence of the present invention.

In the specific embodiment of the present invention, the DNA fragment shown in SEQ ID No. 3 (the gene encoding the heavy chain of the antibody) was cloned between the restriction sites Xba I and Hand III of the pcDNA3.4 vector to obtain a recombinant expression vector for expressing the heavy chain of the antibody (named pcDNA3.4-H). The DNA fragment shown in SEQ ID No. 4 (the gene encoding the light chain of the antibody) was cloned between the restriction sites Xba I and Hand III of the pcDNA3.4 vector to obtain a recombinant expression vector for expressing the light chain of the antibody (named pcDNA3.4-L). In order to be more conducive to protein expression, when constructing the recombinant expression vector, in the upstream of the DNA fragments shown in SEQ ID No. 3 and SEQ ID No. 4 (coding genes for antibody heavy and light chains) a kozak co-recognition sequence (5' -GCCACC-3') and a signal peptide coding sequence (5' -ATGGAGTTTGGGCTGAGTTGGGTCTTTCTGGTCGCAATTCTGCTGAAGG GAGTGCAGTGC-3') are also introduced, and the coding sequence of the signal peptide is connected to the 5'ends of SEQ ID No.3 and SEQ ID No.4. The recombinant cell is a recombinant cell obtained by co-transfecting the aforementioned two recombinant expression vectors (pcDNA3.4-H and pcDNA3.4-L) for expressing the antibody heavy chain and light chain respectively into HEK293 cells. The recombinant cell can express the aforementioned antibody Hanke10F4.

In the seventh aspect, the present invention claims an ADC antibody.

The ADC antibody claimed in the present invention is formed by the aforementioned antibody or the aforementioned antigen-binding fragment or the aforementioned antibody fusion protein (such as a single-chain antibody with a tag protein) or the aforementioned double antibody conjugated with small molecule drug with biological activity.

In the eighth aspect, the present invention claims a pharmaceutical composition.

The pharmaceutical composition claimed in the present invention comprises:
(A1) Aforementioned antibody or aforementioned antigen-binding fragment (e.g. single chain antibody) or aforementioned antibody fusion protein (e.g. single-chain antibody fusion with Fc) or aforementioned double antibody or aforementioned ADC antibody;
(A2) Pharmaceutically acceptable excipients, diluents or carriers.

In the ninth aspect, the present invention claims application of aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition in any of the following:
(B1) Preparation of products for testing 4-1BB, or application in testing 4-1BB;

In practical applications, the antibody or antigen-binding fragment thereof can be used as a diagnostic tool to detect 4-1BB in the blood or tissue of patients with cancer, autoimmune or other diseases.

Wherein, the 4-1BB can be human 4-1BB or monkey 4-1BB.
(B2) Preparation of products for blocking the 4-1BB/4-1BBL signal pathway, or application in blocking the 4-1BB/4-1BBL signal pathway;
(B3) Preparation of products for stimulating T cell activation, or application in stimulating T cell activation;
(B4) Preparation of products for promoting the secretion of IFN-γ by T cells, or application in promoting the secretion of IFN-γ by T cells;
(B5) Preparation of products for inhibiting the growth of colon cancer cells, or application in inhibiting the growth of colon cancer cells;
(B6) Preparation of products for inhibiting the growth of colon cancer tumors, or application in inhibiting the growth of colon cancer tumors;
(B7) Preparation of products for treating and/or preventing colon cancer, or application in treating and/or preventing colon cancer;
(B8) Using as an immune enhancer, or application in preparing an immune enhancer;
(B9) Using an immune modulator, or application in preparing an immune modulator;
(B10) Preparation of products for the treatment and/or prevention and/or diagnosis of diseases characterized by 4-1BB expression dysregulation, or application in the treatment and/or prevention and/or diagnosis of diseases characterized by 4-1BB expression dysregulation.
(B11) Preparation of products for the treatment and/or prevention and/or diagnosis of cancer, or application in the treatment and/or prevention and/or diagnosis of cancer;

Further, the cancer may be a cancer in which the expression of 4-1BB is dysregulated.
(B12) Preparation of products for the treatment and/or prevention and/or diagnosis of autoimmune diseases, or application in treatment and/or prevention and/or diagnosis of autoimmune diseases;

Further, the autoimmune disease may be an autoimmune disease in which the expression of 4-1BB is dysregulated.
(B13) Preparation of products for the treatment and/or prevention and/or diagnosis of inflammatory diseases, or application in the treatment and/or prevention and/or diagnosis of inflammatory diseases;

Further, the inflammatory disease may be an inflammatory disease in which the expression of 4-1BB is dysregulated.
(B14) Preparation of products for the treatment and/or prevention and/or diagnosis of infectious diseases, or application in the treatment and/or prevention and/or diagnosis of infectious diseases.

Further, the infectious disease may be an infectious disease in which the expression of 4-1BB is dysregulated.

In the ninth aspect, the present invention claims any of the following methods:
(C1) A method for detecting 4-1BB, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or aforementioned expression cassette, recombinant vector or recombinant cell to detect the sample to be tested.

In practical applications, the antibody or antigen-binding fragment thereof can be used as a diagnostic tool to detect 4-1BB in the blood or tissue of patients with cancer, autoimmune or other diseases.

Wherein, the 4-1BB can be human 4-1BB or monkey 4-1BB.
(C2) A method for blocking the 4-1BB/4-1BBL signal pathway, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition to block the 4-1BB/4-1BBL signal pathway.
(C3) A method for stimulating T cell activation, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition to stimulate T cell activation.
(C4) A method for promoting the secretion of IFN-γ by T cells, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition to promote the secretion of IFN-γ by T cells.
(C5) A method for inhibiting the growth of colon cancer cells, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or aforementioned expression cassette, recombinant vector or recombinant cell or aforementioned ADC antibody or aforementioned pharmaceutical composition to inhibit the growth of colon cancer cells.
(C6) A method for inhibiting the growth of colon cancer tumors, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition to inhibit the growth of colon cancer tumors.
(C7) A method for treating and/or preventing colon cancer, comprising the following steps: using aforementioned antibody or aforementioned antigen-binding fragment (e.g. single chain antibody) or aforementioned antibody fusion protein (e.g. single-chain antibody fusion with Fc) or aforementioned double antibody aforementioned nucleic acid molecule or expression cassette, recombinant vector or recombinant cell or aforementioned ADC antibody or aforementioned pharmaceutical composition to treat and/or prevent colon cancer.
(C8) A method for preparing an immune enhancer, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition as an active ingredient to prepare an immune enhancer.
(C9) A method for preparing an immune modulator, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition as an active ingredient to prepare an immune modulator.
(C10) A method for treating and/or preventing and/or diagnosing a disease characterized by 4-1BB expression dysregulation, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or aforementioned expression cassette, recombinant vector or recombinant cell or aforementioned ADC antibody or aforementioned pharmaceutical composition to treat and/or prevent and/or diagnose a disease characterized by 4-1BB expression dysregulation.
(C11) A method for treating and/or preventing and/or diagnosing cancer, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition to treat and/or prevent and/or diagnose cancer.

Further, the cancer may be a cancer in which the expression of 4-1BB is dysregulated.
(C12) A method for treating and/or preventing and/or diagnosing autoimmune diseases, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition to treat and/or prevent and/or diagnose autoimmune diseases.

Further, the autoimmune disease may be an autoimmune disease in which the expression of 4-1BB is dysregulated.
(C13) A method for treating and/or preventing and/or diagnosing inflammatory diseases, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition to treat and/or prevent and/or diagnose inflammatory diseases.

Further, the inflammatory disease may be an inflammatory disease in which the expression of 4-1BB is dysregulated.
(C14) A method for treating and/or preventing and/or diagnosing infectious diseases, comprising the following steps: using aforementioned antibody or aforementioned antigen-binding fragment (e.g. single chain antibody) or aforementioned antibody fusion protein (e.g. single-chain antibody fusion with Fc) or aforementioned double antibody or aforementioned nucleic acid molecule or expression cassette, recombinant vector or recombinant cell or aforementioned ADC antibody or aforementioned pharmaceutical composition treat and/or prevent and/or diagnose infectious diseases.

Further, the infectious disease may be an infectious disease in which the expression of 4-1BB is dysregulated.

### Description of the drawings

Figure 1 shows the hybridoma antibody SDS-PAGE.
Figure 2 shows the structure of the antibody.
Figure 3 shows that antibodies secreted by hybridomas recognize human 4-1BB.
Figure 4 shows that antibodies secreted by hybridomas recognize monkey 4-1BB.
Figure 5 shows that the antigen-binding fragments recognize human 4-1BB.
Figure 6 shows that the antigen-binding fragments recognize monkey 4-1BB.
Figure 7 shows the comparison of the affinity of each antigen-binding fragment to human 4-1BB by FACS.
Figure 8 shows the affinity comparison of Hanke10F4 and HKB6.
Figure 9 shows that the antibody Hanke10F4 blocks the binding of 4-1BBL to 4-1BB.
Figure 10 shows the epitope competition of the antibody Hanke10F4 and the control antibodies.
Figure 11 shows the detection of the affinity of antibodies to antigens with different point mutations by FACS.
Figure 12 shows the epitope competition of Hanke10F4 and HKB6 .
Figure 13 shows the assay of luciferase activity; A is the result of "3.5 Luciferase reporter gene activity detection", B is the test result of "3.9 Stability (1) Accelerated Stability".
Figure 14 shows the detection of the effect of antibody Hanke10F4 on activating CD4⁺ T cells; in the figure, three bar charts of each group from left to right are respectively the activation effect of CD4⁺ T cells under the condition of high, medium, and low antibody concentration (2 µ g/ml, 0.4 µ g/ml, 0.08 µ g/ml).
Figure 15 shows the detection of the effect of antibody Hanke10F4 on activating CD8⁺ T cells; in the figure, three bar charts of each group from left to right are respectively the activation effect of CD8⁺ T cells under the condition of high, medium, and low antibody concentration (3 µ g/ml, 0.5 µ g/ml, 0.08 µ g/ml).
Figure 16 is an antibody inhibiting MC38 tumor model; each line represents the tumor growth curve of a mouse.
Figure 17 shows the antibody challenge test of Hanke10F4.
Figure 18 shows the toxicological test of antibody Hanke10F4.
Figure 19 shows the Tm detection of Hanke10F4 .

### Embodiments

The following examples facilitate a better understanding of the present invention, but do not limit the present invention. The experimental methods in the following examples, unless otherwise specified, are all conventional methods. The experimental materials used in the following examples, unless otherwise specified, were all purchased from conventional biochemical reagent stores. The quantitative experiments in the following examples were all set to repeat the experiment three times, and the results were averaged.

In the following examples, the positive control antibodies were Utomilumab (PF-05082566, patent number: US8337850 B2) from Pfizer and Urelumab (BMS-663513, patent number: US7288638 B2) from Bristol Myers Squibb, and both antibodies were anti-human 4-1BB antibodies. The negative control antibody was human IgG (product of Nanjing GenScript, China).

In the following examples, unless otherwise specified, the first position of each nucleotide sequence in the sequence listing was the 5'-terminal nucleotide of the corresponding DNA, and the last position was the 3'-terminal nucleotide of the corresponding DNA.

The pCDNA3.4 vector in the following examples was from Invitrogen, Cat: A14697.

The conventional equipment and reagents were as follows:
1. 96-well microtiter plate (Nunc Company);
2. Plating buffer: phosphate buffer, pH 7.0;
3. Washing solution: Phosphate buffer with pH 7.0 containing only 0.05% by volume of Tween 20;
4. Blocking solution: washing solution containing only 10g/L BSA.
5. Horseradish peroxidase labeled avidin;
6. Chromogenic substrate: Tetramethylbenzidine;
7. Stop solution: 1M sulfuric acid.

Wherein, the solvent of the phosphate buffer with pH 7.0 was water, and the solutes were sodium chloride, potassium chloride, potassium dihydrogen phosphate, and disodium hydrogen phosphate. The concentration of the sodium chloride in the phosphate buffer with pH 7.0 was 135 mM, the concentration of the potassium chloride in the phosphate buffer with pH 7.0 was 2.7 mM, the concentration of the potassium dihydrogen phosphate in the phosphate buffer with pH 7.0 was 1.5 mM, and the concentration of the disodium hydrogen phosphate in the phosphate buffer with pH 7.0 was 8 mM.

### Embodiment 1. Preparation of hybridoma antibody

### 1.1 Mouse Immunization

Three Balb/c mice and three C57b1/6 mice aged 4-6 weeks were used, and the extracellular segment of human 4-1BB (positions 24-186 of SEQ ID No. 5) was used as the antigen to immunize the mice once every 2 weeks for a total of 3 times. The tail vein blood was collected after the third immunization, and the antibody production was detected by indirect ELISA. The appropriate immunized mice were selected, and the mice were sacrificed by cervical dislocation after removal of eyeballs. Anti-human 4-1BB monoclonal antibody hybridoma cell lines were prepared by cell fusion performed according to conventional methods.

### 1.2 Hybridoma screening

The anti-human 4-1BB monoclonal antibody hybridoma cells were screened by ELISA. The ELISA plate was coated with 10µg/ml human 4-1BB (SEQ ID No. 5), and then blocked overnight at 4°C. The cell culture supernatant to be tested was added in sequence, then the ELISA plate was incubated at 37°C for 1 hour, and then washed 3 times with PBST. 4000-fold diluted horseradish peroxidase labeled goat anti-mouse (ThermoFisher company) was added into the plate, and the plate was incubated at 37°C for 45min, washed 3 times with PBST, and after color development with substrate tetramethylbenzidine (TMB) (ThermoFisher), the OD value was measured at a wavelength of 450nm.

The positive cell clones were screened by ELISA and then subcloned repeatedly until all the hybridoma cell culture supernatants were detected as 100% positive. The limiting dilution method was used for the cloning of hybridoma cells. The culture plate was put in a 37°C, 5% CO₂ incubator for culture. After about 5 days, cell clones could be observed under the microscope. The medium was changed at the appropriate time and tested, the positive and well-growing monoclonal cell lines were taken for enlarged culture, then the anti-human 4-1BB monoclonal antibody hybridoma cell lines were obtained and frozen in time.

### 1.3 Identification of hybridoma antibodies

The positive and well-growing anti-human 4-1BB antibody hybridoma cell lines selected were expanded and cultured step by step, centrifugated, and the supernatants were taken and purified with a Protein G affinity chromatography column. The specific operation was: first the Protein G column (GE Company) was equilibrated with PBS, and then the supernatants were passed through the column; solution A (formulation: solvent was water, solutes and concentration were: 20mM sodium phosphate, 500mM NaCl, pH5.0) was used for pre-elution for 5 column volumes; solution B (formulation: solvent was water, solutes and concentration were: 20mM sodium acetate, 150mM NaCl, pH3.5) was used for elution for 5 column volumes; the elution peaks were collected and then concentrated in a 30KDa concentrating centrifuge tube to obtain antibodies.

Fifteen hybridoma antibody cell lines were obtained by immunization. After screening, 6 candidate hybridoma parent cell lines were finally obtained. After subtype identification and preliminary screening, the details of the cell lines and corresponding antibodies are shown in Table 1.

**Table 1. Anti-human 4-1BB hybridoma cell lines**

| No. | Code Name | Antibody subtype | Fusion cell |
|---|---|---|---|
| 1 | 37G10F4 | IgG1,κ | Sp2/0 |
| 2 | 23C2E9 | IgG1,κ | Sp2/0 |
| 3 | 2C2F2 | IgG1,κ | Sp2/0 |
| 4 | 32C2G6 | IgG2b, κ | Sp2/0 |
| 5 | 13F8H8 | IgM, κ | Sp2/0 |
| 6 | 37G11E2B3 | IgM, κ | Sp2/0 |

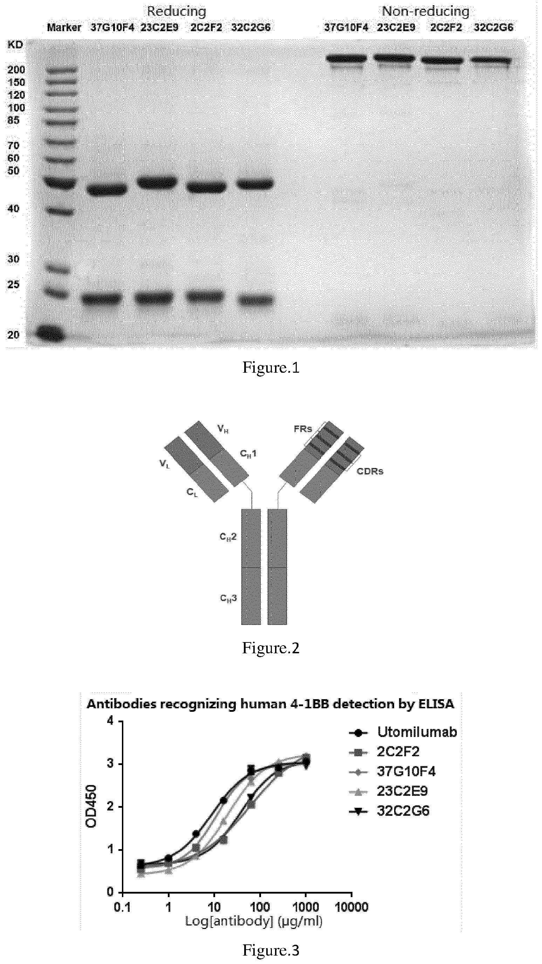

The two candidates, 13F8H8 and 37G11E2B3, were eliminated, the cell supernatants were collected, purified and concentrated respectively, and the samples were subjected to SDS-PAGE electrophoresis. The electrophoresis detection results are shown in Figure 1.

The obtained anti-human 4-1BB antibodies were sequenced, and the results showed that the obtained anti-human 4-1BB antibodies had a molecular weight of 150Kb, including heavy and light chains, and were typical intact antibodies. The structure of the antibodies is shown in Figure 2.

### 1.4 Antibodies secreted by hybridomas recognizing human 4-1BB

The affinity of the antibodies secreted by the four hybridomas to recognize human 4-1BB was evaluated by ELISA. 96-well plate (96-well microtiter plate, Nunc company) was coated with human 4-1BB (SEQ ID No.5) at a concentration of 1 µ g/ml, 100 µ l/well, 4°C overnight. The plate was washed three times, 300 µl of blocking solution was added to each well (see above reagent formula), and the wells were blocked at 37°C for 1 hour. The plate was washed three times with the washing solution (see above for the recipe), and the antibodies were diluted to 4000 ng/ml with the sample diluent (PBS-T with 1% bovine serum albumin), and diluted by four-fold gradient dilution with 7 Ep tubes, two replicate wells were set for each concentration, 100 µ l/well, incubated for 1 hour. The plate was washed three times with washing solution, 100 µ l of horseradish peroxidase labeled goat anti-mouse (ThermoFisher) diluted 8000 times was added to each well, and shaken for 0.5 hour. The plate was washed three times and 100 µ l of tetramethylbenzidine (TMB) (ThermoFisher) was added to each well. Protected from light for color development, 1M sulfuric acid was added to terminate the reaction. The OD value at 450 nm was measured with BIO-TEK ELX-800 microplate reader.

Through the above steps, the antibodies secreted by hybridomas could recognize and bind to human 4-1BB. The OD450 value of the ELISA result is shown in Table 2, and the analysis diagram is shown in Fig. 3.

**Table 2. Antibodies secreted by hybridomas recognizing human 4-1BB**

| Concentration(ng/ml) | Utomilumab | | 32C2G6 | | 23C2E9 | | 2C2F2 | | 37G10F4 | |
|---|---|---|---|---|---|---|---|---|---|---|
| 1000 | 3.071 | 3.057 | 2.974 | 2.942 | 3.232 | 3.169 | 3.174 | 3.142 | 3.092 | 3.036 |
| 250 | 2.927 | 2.912 | 2.913 | 2.988 | 3.038 | 3.072 | 2.813 | 2.788 | 2.975 | 2.941 |
| 62.5 | 2.926 | 2.794 | 2.206 | 2.235 | 2.561 | 2.626 | 2.006 | 2.114 | 2.684 | 2.657 |
| 15.625 | 2.192 | 2.120 | 1.186 | 1.272 | 1.662 | 1.693 | 1.186 | 1.272 | 2.190 | 2.104 |
| 3.9063 | 1.397 | 1.359 | 1.048 | 1.045 | 0.879 | 0.843 | 1.048 | 1.045 | 1.039 | 1.051 |
| 0.9766 | 0.848 | 0.768 | 0.683 | 0.715 | 0.527 | 0.527 | 0.683 | 0.715 | 0.690 | 0.697 |
| 0.2441 | 0.726 | 0.610 | 0.546 | 0.550 | 0.448 | 0.461 | 0.546 | 0.550 | 0.626 | 0.556 |
| 0 | 0.583 | 0.534 | 0.384 | 0.349 | 0.384 | 0.349 | 0.536 | 0.547 | 0.480 | 0.497 |
| EC50(ng/ml) | 8.634 | | 37.06 | | 19.37 | | 60.71 | | 10.68 | |
| R² | 0.996 | | 0.9979 | | 0.9973 | | 0.9985 | | 0.9924 | |

### 1.5 Antibodies secreted by hybridomas recognizing monkey 4-1BB

The affinity of the antibodies secreted by the four hybridomas to recognize monkey 4-1BB was evaluated by ELISA. The monkey 4-1BB (Cy4-1BB) (SEQ ID No.6) antigen was diluted with plating buffer (see above for the recipe) to 1 µ g/ml, 100 µ l was added to each well for plating overnight at 4°C. The antibodies secreted by hybridomas and the positive control antibody Utomilumab were diluted to 1000 ng/ml with the sample diluent, and were 10-fold gradient diluted with 7 Ep tubes, two replicate wells were set for each concentration, and 100 µ l/well was added into the plate, and shaken for 1 hour. The plate was washed again, and 100 µ l of horseradish peroxidase labeled goat anti-mouse (ThermoFisher) diluted 8000 times was added to each well, shaken for 0.5 hour. After washing, 100 µ 1 of tetramethylbenzidine (TMB) (ThermoFisher) was added to develop color.

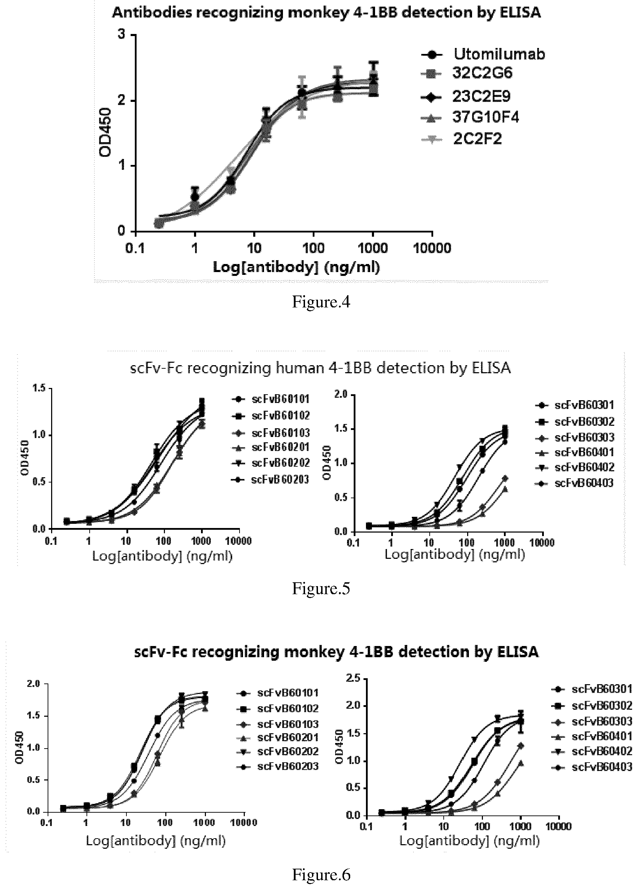

Through the above steps, it was proved that the antibodies secreted by hybridomas could recognize Cy4-1BB molecules by experiments. The OD450 value of the ELISA results is shown in Table 3, and the analysis diagram is shown in Figure 4.

**Table 3. Antibodies secreted by hybridomas recognizing monkey 4-1BB**

| Concentration(ng/ml) | Utomilumab | | 32C2G6 | | 23C2E9 | | 2C2F2 | | 37G10F4 | |
|---|---|---|---|---|---|---|---|---|---|---|
| 1000 | 2.343 | 2.143 | 2.210 | 2.155 | 2.510 | 2.155 | 2.414 | 2.310 | 2.290 | 2.302 |
| 250 | 2.113 | 2.080 | 2.013 | 2.067 | 2.313 | 2.067 | 2.110 | 2.059 | 2.153 | 2.448 |
| 62.5 | 2.107 | 2.144 | 1.922 | 1.965 | 1.922 | 2.165 | 2.272 | 1.835 | 2.110 | 2.071 |
| 15.625 | 1.573 | 1.827 | 1.666 | 1.488 | 1.666 | 1.488 | 1.801 | 1.599 | 1.691 | 1.438 |
| 3.9063 | 0.693 | 0.796 | 0.644 | 0.650 | 0.644 | 0.650 | 0.915 | 0.934 | 0.782 | 0.833 |
| 0.9766 | 0.429 | 0.635 | 0.418 | 0.371 | 0.418 | 0.371 | 0.598 | 0.578 | 0.298 | 0.361 |
| 0.2441 | 0.111 | 0.134 | 0.126 | 0.122 | 0.126 | 0.122 | 0.153 | 0.138 | 0.131 | 0.141 |
| 0 | 0.083 | 0.100 | 0.099 | 0.082 | 0.099 | 0.082 | 0.092 | 0.091 | 0.094 | 0.047 |
| EC50(ng/ml) | 7.394 | | 8.175 | | 9.368 | | 4.969 | | 8.142 | |
| R² | 0.9974 | | 0.9905 | | 0.9813 | | 0.9748 | | 0.992 | |

### Embodiment 2. Humanization of antibodies and identification of the properties of scFv-Fc antigen-binding fragments

The secretory antibody 37G10F4 obtained in the present invention was screened. The antibody gene sequence was analyzed, and the reasonable humanized antibody was designed through a series of analyses such as homology modeling and optimization of antibody Fab, surface scanning to determine humanized mutation sites, virtual mutation and molecular dynamics simulation, and identification of key amino acids. Through humanization, 12 candidate scFv-Fc (human IgG4) antigen-binding fragments (Table 4) were obtained for further identification.

The 12 candidate scFv-Fc (human IgG4) antigen-binding fragments in Table 4 have the same complementarity determining region (CDR) in the variable region of the heavy chain (VH) and the variable region of the light chain (VL) are the same; the only difference is that there are amino acid mutations in the framework region (FR) of VH and VL; and the Fc regions are also the same. The amino acid sequences of HCDR1, HCDR2 and HCDR3 in the variable region of the heavy chain are shown sequentially at positions 31-35, positions 50-64, and positions 98-106 in SEQ ID No. 16 from the N-terminus. The amino acid sequences of LCDR1, LCDR2 and LHCDR3 in the variable region of the light chain are shown sequentially at positions 156-166, positions 182-188, and positions 221-229 in SEQ ID No. 16 from the N-terminus.

Thereinto, the amino acid sequence of scFvB60103 is shown in SEQ ID No. 16, positions 1-117 are the variable region of the heavy chain, positions 133-239 are the variable region of the light chain, and positions 240-472 are Fc.

Corresponding to the gene level, the coding gene sequence of scFvB60103 is shown in SEQ ID No. 17, and positions 1-351 are the coding gene of the heavy chain variable region (positions 91-105, 148-192, and 292-318 respectively encode three HCDR), positions 397-717 are the coding gene of the light chain variable region (positions 466-498, 544-564, and 661-687 respectively encode three LCDRs), and positions 718-1416 are the coding gene for Fc.

The 12 candidate scFv-Fc molecules in Table 4 were expressed and purified. The scFvB60103 was taken as an example. The encoding gene (SEQ ID No. 17) of antibody scFvB60103 was artificially synthesized, and then inserted into the pCDNA3.4 vector and expressed by transient transfection. The transient transfection operation was done following the operating procedures according to the product instructions of Expi293 expression system (Thermo Fisher). Solutions A and B were obtained by adding Expi Fectamine transfection reagent and DNA plasmid into OptiMEM respectively, and then solution C was obtained by mixing solution A and solution B. Solution C was added to Expi293 cells (Thermo Fisher), the Expi293 cells were cultured for 5 days and then centrifuged (10,000 rpm for 10 minutes), the supernatant was taken, and the obtained supernatant was purified with a Protein G affinity chromatography column. The specific operation was: first the Protein G column (GE Company) was equilibrated with PBS, and then the supernatant was passed through the column; first solution A (formulation: solvent was water, solute and concentration: 20mM sodium phosphate, 500mM NaCl, pH5.0) was used for pre-elution for 5 column volumes, then solution B (formulation: solvent was water, solute and concentration: 20mM sodium acetate, 150mM NaCl, pH3.5) was used for elution for 5 column volumes; the elution peaks were collected, and then scFvB60103 target molecules were obtained by concentrating with a 30KDa concentrating centrifuge tube.

### (1) ELISA identification

The affinity of scFv molecules with human and monkey 4-1BB molecules was identified by ELISA (see related steps in Example 1 for specific methods). As shown in Table 4, the results show that all the 12 scFv-Fc molecules can recognize human and monkey 4-1BB, and scFvB60103 has the best recognition ability. The ELISA results are shown in Figure 5 and Figure 6.

**Table 4. ScFv recognizing human and monkey 4-IBB**

| Name | Sequence No. | Subtype | Human EC50(ng/ml) | Monkey EC50(ng/ml) |
|---|---|---|---|---|
| scFvB60101 | SEQ ID No.18 | Human IgG4 | 46.19 | 22.71 |
| scFvB60102 | SEQ ID No.19 | Human IgG4 | 48.81 | 24.47 |
| scFvB60103 | SEQ ID No.16 | Human IgG4 | 44.66 | 21.71 |
| scFvB60201 | SEQ ID No.20 | Human IgG4 | 164.8 | 69.81 |
| scFvB60202 | SEQ ID No.21 | Human IgG4 | 152.8 | 60.82 |
| scFvB60203 | SEQ ID No.22 | Human IgG4 | 82.41 | 36.67 |
| scFvB60301 | SEQ ID No.23 | Human IgG4 | 76.57 | 51.72 |
| scFvB60302 | SEQ ID No.24 | Human IgG4 | 97.47 | 56.56 |
| scFvB60303 | SEQ ID No.25 | Human IgG4 | 46.5 | 26.44 |
| scFvB60401 | SEQ ID No.26 | Human IgG4 | 1042 | 772.4 |
| scFvB60402 | SEQ ID No.27 | Human IgG4 | 618 | 552.7 |
| scFvB60403 | SEQ ID No.28 | Human IgG4 | 194.9 | 107.9 |

### (2) FACS detection

The reagents used included:
FACS buffer, the buffer was a solution obtained by adding BSA and sodium azide to PBS, the mass percentage concentration of BSA in the buffer was 2%, and the mass percentage concentration of sodium azide was 0.02%; Goat anti-human FITC secondary antibody (Sigma, store in the dark).

Using CHO-K1-hu4-1BB, the FACS method was used to detect the difference in affinity of the scFv molecules with better binding activity to human 4-1BB in ELISA test to human 4-1BB molecules. The cells used in the experiment were CHO-K1 cells that highly express human 4-1BB on the cell membrane surface (CHO-K1-hu4-1BB) constructed by China Hefei HankeMab Company, and wild-type CHO-K1 without 4-1BB expression (Shanghai Cell Bank, Chinese Academy of Sciences).

The human 4-1BB gene sequence (SEQ ID No. 7) was artificially synthesized, and then inserted into the pCDNA3.4 vector according to the common method in the field, and then introduced into the wild-type CHO-K1 cells with Lipofectamine 3000 transfection reagent (Invitrogen). Then, G418 (purchased from Sangon Biotech (Shanghai) Co., Ltd.) was added for pressure screening, and finally CHO-K1 cells with high expression of human 4-1BB on the cell membrane (CHO-K1-hu4-1BB) were obtained for use.

The cells to be tested were cultured to 80% fullness with T75 flasks, digested with trypsin, centrifuged at 1000 rpm for 5 minutes, and the cells were collected (the number of cells per flask was about 10⁶). The cells were resuspended and washed with about 1ml buffer, centrifuged and resuspended to obtain a cell suspension with a cell concentration of 1 × 10⁷ cells/ml. 25 µl dilutions of each dilution gradient of each antibody to be tested (see Table 5) were prepared, and 25 µ 1 cell suspension was added to each centrifuge tube with different concentrations of antibodies to mix. The final antibody concentration is shown in Table 5, and the centrifuge tube containing the antibody diluent with the antibody concentration of 0 µ g/ml was used as a blank control. After incubating for 30 minutes, the centrifuge tubes were washed twice with 1ml FACS buffer, added goat anti-human FITC secondary antibody, blown and resuspended, and incubated for 30 minutes in the dark. The centrifuge tubes were washed twice with 1ml FACS buffer again, then added 500 µ 1 FACS buffer to each tube, resuspended, placed on ice and protected from light, and tested on the machine. The antibody Utomilumab (Pfizer Pharmaceuticals Co., Ltd.) was used as a positive control.

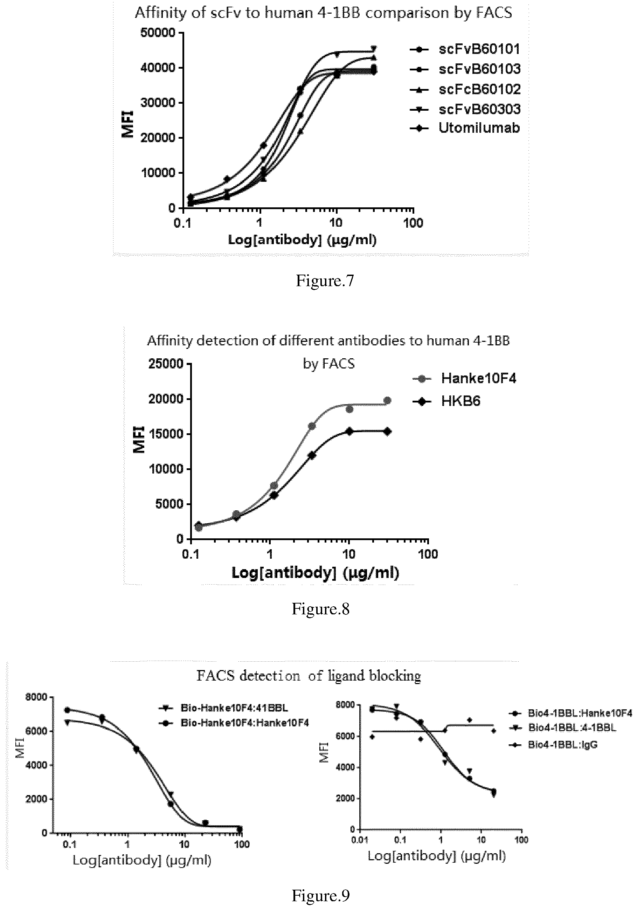

Through the above steps, the binding activity of different scFv molecules with human 4-1BB were compared. As shown in Table 5 and Figure 7, the results show that scFvB60103 has the lowest EC50 value, that is, it has the highest affinity to human 4-1BB, which can be regarded as the best molecule.

**Table 5. FACS detection results of affinity of scFv to human 4-1BB**

| Concentration (µg/ml) | Mean fluorescence intensity | | | | |
|---|---|---|---|---|---|
| | scFvB60101 | scFvB60103 | scFvB60102 | scFvB60303 | Utomilumab |
| 30.00 | 39766.30 | 40475.6 | 43125.1 | 45506.00 | 39051.80 |
| 10.00 | 38200.10 | 38970.3 | 37979.2 | 43806.20 | 38161.50 |
| 3.33 | 26595.70 | 33119 | 22163.1 | 33930.00 | 34092.00 |
| 1.11 | 9754.40 | 11287.9 | 8507.7 | 13871.00 | 18026.80 |
| 0.37 | 3548.80 | 3705.4 | 3360.1 | 4818.40 | 8432.00 |
| 0.12 | 1622.60 | 1775.3 | 1573 | 2311.70 | 3399.50 |
| EC50 | 2.38074 | 1.79679 | 3.50298 | 1.94382 | 1.32403 |
| R² | 0.99955 | 0.99933 | 0.99961 | 0.99985 | 0.99721 |

### Embodiment 3. IgG humanized antibody

The exemplary antibody Hanke10F4 identified in this example was obtained from the humanized modification of the hybridoma secretory antibody 37G10F4, that is, it was transformed from scFvB60103 in Table 4. This embodiment will detail the expression and purification of Hanke10F4, determination with ELISA, FACS and luciferase reporter gene, and identification in T cell activation system.

### 3.1 The expression and purification of antibody

The full-length gene sequence was synthesized according to the gene sequence obtained by sequencing. The gene encoding the heavy chain of the antibody Hanke10F4 is shown in SEQ ID No.3, and the gene encoding the light chain of the antibody Hanke10F4 is shown in SEQ ID No.4.

Positions 1-351 of SEQ ID No. 3 are the coding gene of the variable region of the heavy chain VH. The coding sequences of CDR1, CDR2 and CDR3 are shown in SEQ ID No. 3 at positions 91-105, 148-192, and 292-318, respectively.

Positions 1-321 of SEQ ID No. 4 are the coding gene of the variable region of the light chain VL. The coding sequences of CDR1, CDR2 and CDR3 are shown in SEQ ID No. 4 at positions 70-102, 148-168, and 265-291, respectively.

XbaI recognition sequence(5'-TCTAGA-3'), kozak co-recognition sequence (5'-GCCACC-3') and coding sequence of the signal peptide (5'-ATGGAGTTTGGGCTGAGTTGGGTCTTTCTGGTCGCAATTCTGCTGAAGGGA GTGCAGTGC-3') were added to the 5'ends of SEQ ID No. 3 and SEQ ID No. 4 respectively. The coding sequence of the signal peptide was connected to the 5'ends of SEQ ID No. 3 and SEQ ID No. 4, then terminator (TGA) and HindIII recognition sequences (5'-AAGCTT-3') were added in sequence to the 3'ends of SEQ ID No. 3 and SEQ ID No. 4, respectively. TGA was connected to the 3'ends of SEQ ID No. 3 and SEQ ID No. 4, and the resulting new DNA sequences were marked as heavy chain gene A and light chain gene A, respectively. The heavy chain gene A was synthesized artificially, digested with XbaI and HindIII, connected with the vector backbone obtained by digesting the pCDNA3.4 vector with XbaI and HindIII, the recombinant vector was obtained, and the recombinant vector verified by sequencing was named as pCDNA3.4-H. The light chain gene A was synthesized artificially, digested with XbaI and HindIII, connected with the vector backbone obtained by digesting the pCDNA3.4 vector with XbaI and HindIII, the recombinant vector was obtained, and the recombinant vector verified by sequencing was named as pCDNA3.4-L.

The pCDNA3.4-H and pCDNA3.4-L were introduced into human embryonic kidney cell line HEK293F (ATCC, American Cell Bank) to obtain recombinant cells, and then the recombinant cells were cultured in a 37°C, 5% CO₂ shaking incubator at 120 rpm.

The protein A affinity chromatography column was used to purify the antibody protein from the culture supernatant. The specific operation was: first the Protein A column (GE Company) was equilibrated with PBS, and then the supernatant was passed through the column; first solution A (formulation: solvent was water, solute and concentration: 20mM sodium phosphate, 500mM NaCl, pH5.0) was used for pre-elution for 5 column volumes, then solution B (formulation: solvent was water, solute and concentration: 20mM sodium acetate, 150mM NaCl, pH3.5) was used for elution for 5 column volumes; the elution peaks were collected, and the antibody obtained by concentrating with 30KDa concentrating centrifuge tube was the anti-human 4-1BB antibody.

Sequencing of the anti-human 4-1BB antibody showed that the obtained anti-human 4-1BB antibody was a complete antibody, that was, the antibody was Hanke10F4. This antibody is composed of a heavy chain and a light chain, the amino acid sequence of the heavy chain is shown in SEQ ID No. 1, and the amino acid sequence of the light chain is shown in SEQ ID No. 2. The heavy chain type of Hanke10F4 antibody is IgG4, and the light chain type is kappa chain.

The positions 1-117 of SEQ ID No.1 are the variable region of the heavy chain VH, wherein the sequences of CDR1, CDR2, and CDR3 are shown in positions 31-35, 50-64, and 98-106 of SEQ ID No. 1, respectively.

The positions 1-107 of SEQ ID No.2 are the variable region of the light chain VL, wherein the sequences of CDR1, CDR2, and CDR3 are shown in positions 24-34, 50-56, and 89-97 of SEQ ID No. 2, respectively.

### 3.2 Identification of affinity to human or monkey 4-1BB

### (1) ELISA detection

For the method, refer to step 1.4 in Example 1. The 96-well plate was directly coated with human 4-1BB or monkey 4-1BB, respectively. Antibody samples (Hanke10F4 and Utomilumab) were used as primary antibodies, and horseradish peroxidase-labeled goat anti-mouse was used as secondary antibody. Tetramethylbenzidine (TMB) was used as a color reagent, and sulfuric acid was used to stop the color development. The OD value was measured at 450 nm with BIO-TEK ELX-800 microplate reader. Table 6 shows the ELISA results of the antibody Hanke10F4 in the form of IgG4, which can recognize both human 4-1BB and monkey 4-1BB.

**Table 6. Binding of HankelOF4 in ELISA assay**

| Antibody concentration ( ng/mL) | Human | | | | Monkey | |
|---|---|---|---|---|---|---|
| | HankelOF4 | | Utomilumab | | HankelOF4 | |
| 1000 | 1.876 | 1.884 | 2.045 | 1.997 | 1.843 | 1.827 |
| 250 | 1.906 | 1.830 | 1.937 | 1.834 | 1.817 | 1.820 |
| 62.5 | 1.549 | 1.516 | 1.905 | 1.791 | 1.401 | 1.464 |
| 15.625 | 0.871 | 0.864 | 1.519 | 1.463 | 0.653 | 0.668 |
| 3.90625 | 0.35 | 0.343 | 0.519 | 0.515 | 0.252 | 0.256 |
| 0.9765625 | 0.153 | 0.179 | 0.196 | 0.192 | 0.105 | 0.103 |
| 0.2441406 | 0.101 | 0.116 | 0.114 | 0.102 | 0.063 | 0.062 |
| 0 | 0.086 | 0.081 | 0.077 | 0.083 | 0.050 | 0.049 |
| EC50 (ng/mL) | 8.068 | | 18.55 | | 26.71 | |
| R² | 0.9959 | | 0.9936 | | 0.9987 | |

### (2) FACS detection

The reagents used included:
FACS buffer, the buffer was a solution obtained by adding BSA and sodium azide to PBS, the mass percentage concentration of BSA in the buffer was 2%, and the mass percentage concentration of sodium azide was 0.02%; Goat anti-human FITC secondary antibody (Sigma, store in the dark).

The cells used in the experiment were CHO-K1 cells that highly express human 4-1BB on the cell membrane surface (CHO-K1-hu4-1BB) and CHO-K1 cells that highly express monkey 4-1BB on the cell membrane surface (CHO-K1-cy4-1BB) constructed by China Hefei HankeMab Company, and wild-type CHO-K1 without 4-1BB expression (Shanghai Cell Bank, Chinese Academy of Sciences).

The human 4-1BB gene sequence (SEQ ID No.7) and the monkey 4-1BB gene sequence (SEQ ID No.8) were artificially synthesized, and then inserted into the pCDNA3.4 vector according to the common method in the field, and then introduced into the wild-type CHO-K1 cells with Lipofectamine 3000 transfection reagent (Invitrogen). Then, G418 (purchased from Sangon Biotech (Shanghai) Co., Ltd.) was added for pressure screening, and finally CHO-K1 cells with high expression of human 4-1BB on the cell membrane (CHO-K1-hu4-1BB) and high expression of monkey 4-1BB on the cell membrane (CHO-K1-cy4-1BB) were obtained.

CHO-K1-hu4-1BB was used to detect the binding activity of Hanke10F4 monoclonal antibody to human 4-1BB. The cells to be tested were cultured to 80% fullness with T75 flasks, digested with trypsin, centrifuged at 1000 rpm for 5 minutes, and the cells were collected (the number of cells per flask was about 10⁶). The cells were resuspended and washed with about 1ml buffer, centrifuged and resuspended to obtain a cell suspension with a cell concentration of 1 × 10⁷ cells/ml. 25 µ l dilutions of each dilution gradient of antibody Hanke10F4 were prepared, and 25 µ 1 cell suspension was added to each centrifuge tube with different concentrations of antibody to mix. The final antibody concentration is shown in Table 7, and the centrifuge tube containing the antibody diluent with the antibody concentration of 0 µ g/ml was used as a blank control. After incubating for 30 minutes, the centrifuge tubes were washed twice with 1ml FACS buffer, added goat anti-human FITC secondary antibody, blown and resuspended, and incubated for 30 minutes in the dark. The centrifuge tubes were washed twice with 1ml FACS buffer again, then added 500 µ l FACS buffer to each tube, resuspended, placed on ice and protected from light, and tested on the machine. The antibody Utomilumab (Pfizer Pharmaceuticals Co., Ltd.) was used as a positive control.

According to the above steps, CHO-K1-cy4-1BB was used to detect the binding activity of Hanke10F4 monoclonal antibody to monkey 4-1BB.

Through the above steps, experiments proved that the Hanke10F4 monoclonal antibody has high binding activity to human 4-1BB and monkey 4-1BB molecules, as shown in Table 7.

**Table 7. FACS detection results of 4-1BB antibody affinity**

| Antibody concentration | Average FITC signal intensity | | Antibody concentration | Average FITC signal intensity | |
|---|---|---|---|---|---|
| (µg/mL) | Human | | (µg/mL) | Monkey | |
| - | HankelOF4 | Utomilumab | -- | HankelOF4 | Utomilumab |
| 30 | 15737.3 | 17078.3 | 6 | 7374.5 | 6537.7 |
| 10 | 16863.6 | 16440.7 | 2 | 5020.8 | 4910.7 |
| 3.333333 | 13644.8 | 16027.1 | 0.666667 | 2360.5 | 2560.2 |
| 1.111111 | 5730.2 | 6681.1 | 0.222222 | 1461 | 1579 |
| 0.37037 | 2866.6 | 3741.6 | 0.074074 | 1390.5 | 1468.2 |
| 0.123457 | 1925.7 | 2270.3 | 0.024691 | 1380.4 | 1395.3 |
| EC50 (µg/mL) | 1.77445 | 1.49076 | EC50 (µg/mL) | 1.80568 | 1.4742 |
| R² | 0.9999 | 0.9971 | R² | 0.99 | 0.99 |

### (3) Comparison of antibody affinities by FACS

CHO-K1-hu4-1BB was used to test and compare the binding activity of Hanke10F4 and HKB6 monoclonal antibodies with human 4-1BB. The cells to be tested were cultured to 80% fullness with T75 flasks, digested with trypsin, centrifuged at 1000 rpm for 5 minutes, and the cells were collected (the number of cells per flask was about 10⁶). The cells were resuspended and washed with about 1ml buffer, centrifuged and resuspended to obtain a cell suspension with a cell concentration of 1 × 10⁷ cells/ml. 25 µ l dilutions of each dilution gradient of antibody Hanke10F4 or HKB6 were prepared, and 25 µ 1 cell suspension was added to each centrifuge tube with different concentrations of antibody to mix. The final antibody concentration is shown in Table 7, and the centrifuge tube containing the antibody diluent with the antibody concentration of 0 µ g/ml was used as a blank control. After incubating for 30 minutes, the centrifuge tubes were washed twice with 1ml FACS buffer, added goat anti-human FITC secondary antibody, blown and resuspended, and incubated for 30 minutes in the dark. The centrifuge tubes were washed twice with 1ml FACS buffer again, then added 500 µ 1 FACS buffer to each tube, resuspended, placed on ice and protected from light, and tested on the machine.

Wherein, HKB6 was the HKB6 antibody described in the patent application CN201811541545.8.

Through the above steps, as shown in Table 8 and Figure 8, the affinity of Hanke10F4 to human 4-1BB is significantly higher than that of HKB6.

**Table 8. Comparison of affinity of Hankering 10F4 and HKB6**

| Antibody concentration (µg/mL) | Average FITC signal intensity | |
|---|---|---|
| | Human | |
| | HankelOF4 | HKB6 |
| 30 | 19887.30 | 15444.9 |
| 10 | 18616.10 | 15465.9 |
| 3.333333 | 16224.80 | 12021.9 |
| 1.111111 | 7719.80 | 6350 |
| 0.370370 | 3678.60 | 3221.9 |
| 0.123457 | 1705.50 | 2026.6 |
| EC50 (µg/mL) | 1.59737 | 1.79677 |
| R² | 0.99 | 0.99 |

### (4) Determination of antibody affinity

The biofilm layer interference (BLI) technology was used to detect the affinity of Utomilumab, Hanke10F4 or Urelumab with the h4-1BB-His antigen(Acro company, product number: Q07011-1) and the Fortebio octec instrument. The results are shown in Table 9. Hanke10F4 has a high antibody affinity, which is comparable to that of Utomilumab.

**Table 9. Antibody affinity**

| Samples | KD(M) | ka (1/Ms) | kd (1/s) |
|---|---|---|---|
| HankelOF4 | 1.39E-08 | 1.73E+05 | 2.41E-03 |
| Utomilumab | 2.21E-08 | 2.43E+05 | 5.38E-03 |
| Urelumab | 5.76E-09 | 2.83E+05 | 1.63E-03 |

| | | | |
|---|---|---|---|
| Note: ka represents the generation rate of the binding product per unit time; kd represents the percentage of the binding product degraded to reactant per unit time; KD value represents the strength of the affinity. | | | |

### 3.3 Ligand competition

CHO-K1-hu4-1BB was used to compare the competition binding activity between antibody Hanke10F4 and human 4-1BBL. The secondary antibody was SA-FITC (Biolegend), and other operations (except the following) and diluents were the same as the FACS detection in step 3.2. The amino acid sequence of human 4-1BBL is shown in SEQ ID No.9.

The Hanke10F4 was labeled as Bio-Hanke10F4 with biotin, and the labeling method was the same as the conventional protein labeling method in the art. The Bio-Hanke10F4 was diluted to solution A with a concentration of 1.5 µ g/ml with the sample diluent. At the same time, diluent B of human 4-1BBL-hIgG (or Hanke10F4) was prepared, the concentration of solution A was fixed, and the concentration of solution B was a certain multiple of solution A (see Table 9 for specific multiples). Solution A and Solution B was mixed in equal proportions and incubated with CHO-K1-hu4-1BB.

The same operation was performed to detect reverse competition, human 4-1BBL was labeled as Bio-4-1BBL with biotin, then was diluted to be solution A with a concentration of 2 µg/ml with the sample diluent. At the same time, diluent B of Hanke10F4 (or human 4-1BBL-hIgG) was prepared, the concentration of solution A was fixed, and the concentration of solution B was a certain multiple of solution A (see Table 8 for specific multiples). Solution A and Solution B was mixed in equal proportions and incubated with CHO-K1-hu4-1BB.

Through the above steps, the experiment proved that the Hanke10F4 monoclonal antibody has a competitive relationship for the binding site of human 4-1BBL. The specific results are shown in Table 10 and Figure 9, that is, the monoclonal antibody will block the human 4-1BB/4-1BBL signaling pathway to a certain extent with the dose of the drug.

**Table 10. Ligand blocking detection by FACS**

| A:Bio-HankelOF4 (1.5µg/ml) | | | A:Bio-4-1BBL (2µg/ml) | | |
|---|---|---|---|---|---|
| (B:A) Ratio | 41BBL | HankelOF4 | (B:A) Ratio | 41BBL | HankelOF4 |
| 60 | 254.3 | 230.2 | 40 | 3220.6 | 3506.1 |
| 15 | 584.3 | 634.6 | 10 | 3772.6 | 4311.4 |
| 3.75 | 2292.8 | 1744.9 | 2.5 | 6319.5 | 5863.4 |
| 0.9375 | 4861.7 | 5000.5 | 0.625 | 6845.4 | 6922.6 |
| 0.23437 | 6581.5 | 6839.2 | 0.15625 | 7911 | 7455.2 |
| 0.058593 | 6311.7 | 7263.8 | 0.03906 | 7740.6 | 7703.4 |
| R² | 0.9954 | 0.9982 | R² | 0.9711 | 0.9983 |

### 3.4 Epitope competition

### 3.4.1 Antigen epitope competition between Hanke10F4 and the positive control antibodies

CHO-K1-hu4-1BB was used to compare the epitope competition relationship between antibody Hanke10F4 and the control antibodies Utomilumab and Urelumab. The secondary antibody was SA-FITC (Biolegend), and other operations (except the following) and diluents were the same as the FACS detection in step 3.2.

The Hanke10F4 was labeled as Bio-Hanke10F4 with biotin, then was diluted to solution A with a concentration of 1.5 µ g/ml with the sample diluent. At the same time, diluent B of antibodies Utomilumab, Urelumab and Hanke10F4 were prepared. The concentration of solution A was fixed, and the concentration of solution B was a certain multiple of solution A (see Table 10 for specific multiples). Solution A and Solution B was mixed in equal proportions and incubated with CHO-K1-hu4-1BB.

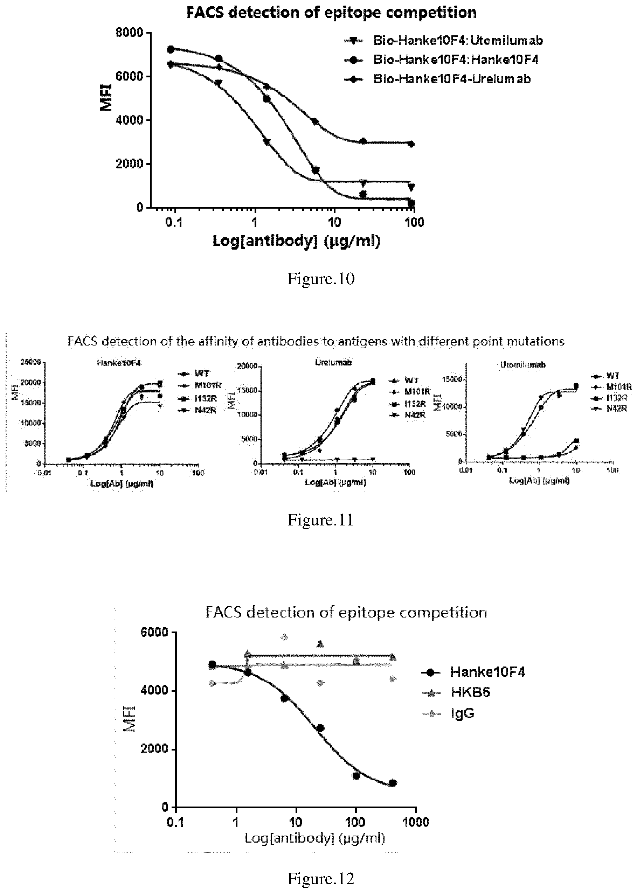

Through the above steps, repeated experiments proved that the Hanke10F4 monoclonal antibody has a certain degree of competition relationship with the antigen binding sites of Utomilumab and Urelumab, and the competition with Utomilumab is more ferocious than with Urelumab. The specific results are shown in Table 11 and Figure 10.

**Table 11. Epitope competition detection by FACS**

| A:Bio-HankelOF4 (1.5µg/ml) | | | |
|---|---|---|---|
| (B: A) Ratio | Utomilumab | Urelumab | HankelOF4 |
| 60 | 934 | 2920 | 230.2 |
| 15 | 1120.8 | 3076.9 | 634.6 |
| 3.75 | 1680.6 | 3961.9 | 1744.9 |
| 0.9375 | 2979.1 | 5543 | 5000.5 |
| 0.234375 | 5694.4 | 6457 | 6839.2 |
| 0.05859375 | 6530.1 | 6592.8 | 7263.8 |
| R² | 0.9909 | 0.9984 | 0.9982 |

### 3.4.2 Antigen epitope analysis of Hanke10F4 and the control antibodies (1) Method I

The extracellular domains of human 4-1BB antigen were replaced with the corresponding extracellular domains of mouse 4-1BB, different target genes were synthesized and inserted into pCDNA3.4 vector to obtain DNA plasmids of different 4-1BB antigens. Cell lines expressing different 4-1BB antigens on the cell surface were obtained by transient transfection, as shown in Table 12, which were used to analyze the binding activity of antibodies to different 4-1BB antigens, and then analyze the specific epitopes of antibody binding to the extracellular domains of human 4-1BB.

Expi293 expression system products (Thermo Fisher) were specifically used for the transient transfection operation, and the operating procedures were based on the product instruction. Solutions A and B were obtained by adding Expi Fectamine transfection reagent and DNA plasmid into OptiMEM respectively, and then solution C was obtained by mixing solution A and solution B. Solution C was added to Expi293 cells (Thermo Fisher), and incubated overnight to obtain a cell line that highly expressed 4-1BB antigen on the cell surface.

Wherein, the full-length amino acid sequence of the human 4-1BB antigen is shown in SEQ ID No. 5 (the corresponding coding gene is shown in SEQ ID No. 7), and the extracellular domain is at positions 24-186 (the corresponding coding gene is at positions 70-558 of SEQ ID No.7). The full-length amino acid sequence of the mouse 4-1BB antigen is shown in SEQ ID No. 10 (the corresponding coding gene is shown in SEQ ID No. 11), and the extracellular domain is at positions 24-187 (the corresponding coding gene is at positions 70-561 of SEQ ID No.11).

Following the FACS detection procedure in step 3.2, antibody samples (Utomilumab, Hanke10F4 or Urelumab) were added in sequence into the cell lines obtained by transient transfection as the primary antibody and incubated with the cells. Then goat anti-human FITC was added as the secondary antibody, incubated, and washed with FACS buffer twice again. Then 500µl FACS buffer was added to each tube, resuspended, protected from light, and tested on the machine.

According to the analysis of patents and literature reports, Utomilumab mainly binds to Domain 3 and Domain 4 of the extracellular domain of human 4-1BB, and Urelumab mainly binds to Domain 1 of the extracellular domain of human 4-1BB. Through the above operations, it could be known that the fluorescence intensity trend of Urelumab decreased in the two groups of cells A and F, and that of Utomilumab decreased in the three groups of cells B, C, and D, which was in line with the research report. The fluorescence intensity trend of Hanke10F4 decreased in the two groups of cells A and F, and greatly decreased in the cell A group, and the degree of decrease was much lower than that of Urelumab. And because the fluorescence intensity of Hanke10F4 did not decrease but increased in cell B group, it showed that Domain1 and 2A were critical to the affinity of Hanke10F4 and human 4-1BB, while Domain2B, 3A, and 3B had little effects. Hanke10F4 mainly binded to the Domain 1 and Domain 2A of the extracellular domains of human 4-1BB, different with the antigen binding epitopes of the control antibodies Utomilumab and Urelumab (Table 12). Hanke10F4 had a certain degree of competition relationship with the positive control antibodies, which might be caused by the spatial epitope of the antigen and antibody.

**Table 12. Analysis of the Mean fluorescence intensity of antibody binding to human 4-1BB epitope**

| Cell number | Specific combination structure of antigen | Urelumab | Utomilumab | Hanke10F4 |
|---|---|---|---|---|
| WT | Human 4-1BB full length | 145990.6 | 325247.2 | 288028.1 |
| A | Human 4-1BB + Mouse 4-1BB domain 1,2 A, 2B (aa24-86) | 1820.8 (decreased) | 19042.1 | 947.7 (decreased) |
| B | Human 4-1BB + Mouse 4-1BB domain2B,3A,3B(aa64-118) | 313213.4 | 5162.1 (decreased) | 466582.9 |
| C | Human 4-1BB + Mouse 4-1BB domain3A,3B,4A(aa87 -133) | 193453.8 | 2310.7 (decreased) | 400516.3 |
| D | Human 4-1BB + Mouse 4-1BB domain3B,4A,4B(aa97-159) | 123207 | 2077.7 (decreased) | 289967.9 |
| E | Human 4-1BB + Mouse 4-1BB domain4A,4B ,NF(aa119-186) | 193919.1 | 100555.4 | 298385.9 |
| F | Human 4-1BB + Mouse 4-1BB domain 1,4B ,NF(aa24-46,aa139-186) | 4906.2 (decreased) | 12583.4 | 1874 (decreased) |

| | | | | |
|---|---|---|---|---|
| Note: The specific structure of the antigen in cell A "Human 4-1BB + Mouse 4-1BB domain1,2A, 2B (aa24-86)" refers to the amino acids at positions 24-86 in the full length of the human 4-1BB antigen as shown in SEQ ID No.5 are replaced with the amino acids at positions 24-86 in the full length of the mouse 4-1BB antigen as shown in SEQ ID No.10. The specific structure of the antigen in cell B "Human 4-1BB + Mouse 4-1BB domain2B,3A,3B(aa64-118)" refers to the amino acids at positions 64-118 in the full length of the human 4-1BB antigen as shown in SEQ ID No.5 are replaced with the amino acids at positions 64-118 of SEQ ID No.10. The specific structure of the antigen in cell C "Human 4-1BB + Mouse 4-1BB domain3A,3B,4A(aa87-133)" refers to the amino acids at positions 87-133 in the full length of the human 4-1BB antigen as shown in SEQ ID No.5 are replaced with the amino acids at positions 87-133 of SEQ ID No.10. The specific structure of the antigen in cell D "Human 4-1BB + Mouse 4-1BB domain3B,4A,4B(aa97-159)" refers to the amino acids at positions 97-159 in the full length of the human 4-1BB antigen as shown in SEQ ID No.5 are replaced with the amino acids at positions 97-159 of SEQ ID No.10. The specific structure of the antigen in cell E "Human 4-1BB + Mouse 4-1BB domain4A,4B,NF(aa119-186)" refers to the amino acids at positions 119-186 in the full length of the human 4-1BB antigen as shown in SEQ ID No.5 are replaced with the amino acids at positions 119-186 of SEQ ID No.10. The specific structure of the antigen in cell F "Human 4-1BB + Mouse 4-1BB domain1,4B,NF(aa24-46,aa139-186)" refers to the amino acids at positions 24-46 and 139-186 in the full length of the human 4-1BB antigen as shown in SEQ ID No.5 are replaced with the amino acids at positions 24-46 and 139-186 of SEQ ID No.10. | | | | |

### (2) Method II

According to literature analysis, the amino acids at positions 101 and 132 of the human 4-1BB antigen are the key binding sites of Utomilulab, and position 42 is the key binding site of Urelumab. The amino acids at the above positions were mutated one by one to synthesize different target genes, DNA plasmids with different 4-1BB antigens were obtained, and transfected transiently to obtain cell lines expressing different 4-1BB antigens on the cell surface (refer to step (1) for related steps), specific as shown in Table 13.

**Table 13. Cells transiently transfected with human 4-1BB point mutations**

| Cell number | Antigen Information |
|---|---|
| WT | Full length of Human 4-IBB |
| M101R | Mutation of amino acid M at position 101 of human 4-IBB to R |
| I132R | Mutation of amino acid I at position 132 of human 4-IBB to R |
| N42R | Mutation of amino acid N at position 42 of human 4-IBB to R |

The cell lines obtained by transient transfection were used, following the FACS detection procedure in step 3.2, antibody Hanke10F4 was added and incubated with the cells. Then goat anti-human FITC was added as the secondary antibody, incubated, and washed with FACS buffer twice again. Then 500µl FACS buffer was added to each tube, resuspended, protected from light, and tested on the machine to analyze the binding activity of antibodies to different 4-1BB antigens. The result is shown in Figure 11. Both Utomilumab and Urelumab did not bind to the antigens with key amino acid mutations, while there was no significant difference in binding ability of Hanke10F4 to all the above antigens, and there was no significant decline. That is to say, the key amino acids that Hanke10F4 binds to the antigen are inconsistent with antibodies Utomilumab and Urelumab, and Hanke10F4 has a specific and unique antigen binding site.

### 3.4.3 Epitope competition of Hanke10F4 and HKB6 binding to antigen

CHO-K1-hu4-1BB was used to compare the antigen-binding epitope competition between antibody Hanke10F4 and HKB6. The secondary antibody was SA-FITC (Biolegend), and other operations (except the following) and diluents were the same as the FACS detection in step 3.2.

The Hanke10F4 was labeled as Bio-Hanke10F4 with biotin, then was diluted to solution A with a concentration of 1.5 µ g/ml with the sample diluent. At the same time, diluent B of antibodies HKB6 and Hanke10F4 were prepared. The concentration of solution A was fixed, and the concentration of solution B was a certain multiple of solution A (see Table 14 for specific multiples). Solution A and Solution B was mixed in equal proportions and incubated with CHO-K1-hu4-1BB.

Through the above steps, the experiment proved that the Hanke10F4 monoclonal antibody does not have a competitive relationship with HKB6, that is, the antigen binding epitopes of the two antibodies are different. The specific results are shown in Table 14 and Figure 12.

**Table 14. Epitope competition of HankelOF4 and HKB6**

| A:Bio-HuB6 (1.5ug/ml) | | | |
|---|---|---|---|
| (B: A) Ratio | HankelOF4 | HKB6 | IgG |
| 60 | 254.3 | 230.2 | 230.2 |
| 15 | 857 | 5189.9 | 4423.9 |
| 3.75 | 1104.7 | 5037.6 | 5052.1 |
| 0.9375 | 2731.3 | 5628.9 | 4293.7 |
| 0.234375 | 3762 | 4895 | 5854.5 |
| 0.05859375 | 4641.7 | 5298.6 | 4847.5 |
| R² | 0.9929 | NA | NA |

### 3.5 Luciferase reporter gene activity detection

HEK-293/NFκB-Luci/41-BB cells are HEK293 cell lines constructed by China Hefei HankeMab Biotechnology Company that stably express human 4-1BB membrane protein and NPκB-Luciferase reporter gene. The specific operations were as follows: the human 4-1BB sequence (SEQ ID No. 7) was inserted as the target gene into the vector pCDNA3.4 to obtain plasmid A, meanwhile the NFκB element sequence (SEQ ID No. 12) and the luciferase gene (SEQ ID No. 13) were inserted as target genes into different positions in the vector pGL4.10 (Youbio company, China) according to the common method in the field to obtain Plasmid B. Then Lipofectamine 3000 transfection reagent (Invitrogen) was used to introduce the A and B plasmids into HEK293 cells (Shanghai Cell Bank, Chinese Academy of Sciences). Then Puromycin (Gibco) and Hygromycin B (Sangon Biotech (Shanghai) Co., Ltd.) were added for pressure screening, and finally HEK-293/NFκB-Luci/41-BB was obtained.

Pressurized medium (DMEM+10%FBS+1%P/S+1ug/ml Puromycin+ 250ug/ml Hygromycin B) was prepared, the HEK-293/NFκB-Luci/41-BB cells were maintained and cultivated in a static incubator with 5% CO₂ at 37°C. The cells in the logarithmic growth phase were taken and centrifuged at 1000 RPM for 5 min after trypsinization, and resuspended in complete medium (DMEM+10%FBS+1%P/S). 50 µ 1 of cells were plated in a 96-well plate (Corning, 3917), 3 × 10⁴ cells/well. The antibody and the cross-linked antibody Fab' goat anti-human IgG Fc (Jackson, 109-006-008) were mixed at a concentration ratio of 1:2, then added into the 96-well plate, 50 µ l/well. After standing for 18-24h in the incubator, 100 µ l of ONE-Glo Luciferase assay sysytem reagent (Promega) was added into the 96-well plate, then incubated for 10min at room temperature, and the chemiluminescence value was detected.

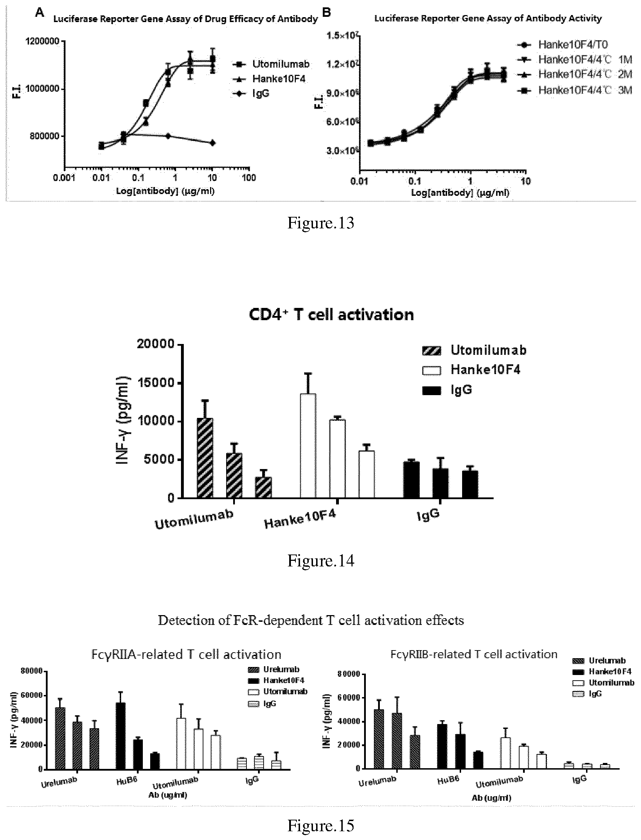

Through the above steps, the experiment proved that both the Hanke10F4 monoclonal antibody and Utomilumab can activate the reporter gene expressing signal. The specific results are shown in Figure 13A. This shows that both Hanke10F4 and Utomilumab can activate the T cell activation signaling pathway and initiate T cell immune function. The activation effect of Hanke10F4 is significantly higher than that of Utomilumab.

### 3.6 In vitro efficacy

### 3.6.1 CD4⁺ T cell activation effect detection

The anti-CD3 antibody (Biolegend, catalog number 317325) was diluted with PBS to 1 µg/ml to obtain the anti-CD3 antibody solution. The antibody Hanke10F4 was taken at three concentrations of high, medium, and low, and diluted with PBS to obtain solutions with Hanke10F4 antibody concentrations of 4 µ g/ml, 0.4 µ g/ml, and 0.04 µ g/ml, respectively. The anti-CD3 antibody solution was mixed with the three concentrations of Hanke10F4 antibody solution at a volume ratio of 1:1 respectively, then added into the 96-well plate, 50 µ l/well, 3 replicates per well, and the 96-well plate was statically incubated at 37°C for 1 hour. The plate was washed three times with PBS before use the next day. Utomilumab was used as the positive control antibody, and IgG (Biolegend, catalog number 403601) was used as the negative control antibody.

Isolation of human CD4⁺ T cells: whole blood was collected from healthy people, lymphocyte separation solution (Sigma) was used to separate PBMC cells according to its product manual. Human CD4⁺ T cell magnetic beads (BD company, catalog number 557767) were used to separate and purify human CD4⁺ T cells according to the product manual for use.

CD4⁺ T cells were added into the 96-well cell culture plate which was incubated at 37°C and coated, with density of 1×10⁵ cells/well. The 96-well plate was incubated in a 5% CO₂ incubator at 37°C for 3 days. The cell supernatant was collected, and the secretion of cytokine INF- γ was detected by ELISA.

Through the above steps, experiments proved that Hanke10F4 antibody is similar to Utomilumab, and both can stimulate CD4⁺ T cell activation and secrete cytokine INF-γ. The specific results are shown in Figure 14.

### 3.6.2 CD8⁺ T cell activation effect detection

The antibody anti-CD3 was diluted to 0.5µg/ml with PBS, then added to a 96-well plate (Corning), 60µl/well, and the 96-well plate was incubated at 37°C for 1 hour. The plate was washed with PBS, and then added the CHO-K1 stable cell line (CHO-K1-CD32A or CHO-K1-CD32B or CHO-K1), 100 µ l/well, 1 × 10⁴ cells/well. Then the 96-well plate was put in the cell incubator overnight.

The cells used in the experiment were CHO-K1 cells (CHO-K1-CD32A) with high expression of human FcγRIIA (CD32A) on the cell membrane constructed by China Hefei HankeMab Company and CHO-K1 cells (CHO-K1-CD32B) with high expression of human FcγRIIB (CD32B) on the cell membrane surface and CHO-K1 cells.

The coding gene sequence of human FcγRIIA (SEQ ID No.14) and the coding gene sequence of human FcγRIIB (SEQ ID No.15) were artificially synthesized, and then inserted into the vector pCDNA3.4 respectively according to common methods in the art, and then introduced into wild-type CHO-K1 cells with Lipofectamine 3000 transfection reagent (Invitrogen) respectively. Then, G418 (purchased from Sangon Biotech (Shanghai) Co., Ltd.) was added for pressure screening, and finally CHO-K1 cells (CHO-K1-CD32A) with high expression of human FcγRIIA on the cell membrane and CHO-K1 cells (CHO-K1-CD32B) with high expression of human FcγRIIB on the cell membrane were obtained.

Isolation of human CD8⁺ T cells: whole blood was collected from healthy people, lymphocyte separation solution (Sigma) was used to separate PBMC cells according to its product manual. Human CD8⁺ T cell magnetic beads (BD company, catalog number 557766) were used to separate and purify human CD8⁺ T cells according to the product manual for use.

After incubating the 96-well plate overnight, the cell supernatant was aspirated, and then 100 µl of human CD8⁺ T cells were added to each well, 1×10⁵ cells/well. Then, the antibody was diluted with medium to the target concentration and then added to a 96-well plate, 100 µ l/well, the final concentration were: 3 µ g/ml, 0.5 µ g/ml, 0.08 µ g/ml, 3 replicate wells were set for each concentration. The 96-well plate was incubated in a cell incubator for 3 days, and the secretion of cytokine INF-γ in the cell culture supernatant was detected by ELISA.

Through the above steps, the experiments proved that the antibody Hanke10F4 is similar to Utomilumab and Urelumab, all of them can stimulate T cell activation, and the activation of T cells depends on FcγRIIA and FcγRIIB. The specific results are shown in Figure 15.

### 3.7 In vivo efficacy

In the experiment, B-hCD137 (4-1BB) mice were selected to detect the anti-tumor efficacy of Hanke10F4 in vivo. The B-hCD137 (4-1BB) mouse model is a genetically engineered mouse with a human-derived h4-1BB gene that is chimeric in the genetic background, genome of C57BL/6 mouse, from Biocytogen Pharmaceuticals (Beijing) Co., Ltd, China.

The MC38 cell line was inoculated subcutaneously on one side of the back (shaved) of the test mouse (each mouse was inoculated with 2 × 10⁶ cells, the cells were mixed with Matrigel at a volume ratio of 1:1 and then inoculated, 200 µ l). When the average tumor volume of the tumor-bearing mice reached 100mm³, the mice were randomly divided into 5 groups according to the experimental design, with 8 mice in each group. The day of group administration was defined as day 0. The grouping situation and dosing schedule are shown in Table 15:

**Table 15. Grouping and dosing schedule**

| Gro up | Test substance | N | Drug dosage (mg/kg) | Route of administration | Dosing frequency |
|---|---|---|---|---|---|
| 1 | Vehicle | 8 | N/A | i.p. | BIW, ^{∗}3 weeks |
| 2 | HankelOF4 high dose | 8 | 3 | i.p. | BIW, ^{∗}3 weeks |
| 3 | Hanke10F4 medium dose | 8 | 1 | i.p. | BIW, ^{∗}3 weeks |
| 4 | HankelOF4 low dose | 8 | 0.3 | i.p. | BIW, ^{∗}3 weeks |
| 5 | Utomilumab | 8 | 1 | i.p. | BIW, ^{∗}3 weeks |

| | | | | | |
|---|---|---|---|---|---|
| Note: N is the number of animals in each group | | | | | |

After tumor inoculation, the survival and activities of the animals were checked twice a week, including tumor growth, mobility, diet, weight and other abnormal behaviors.

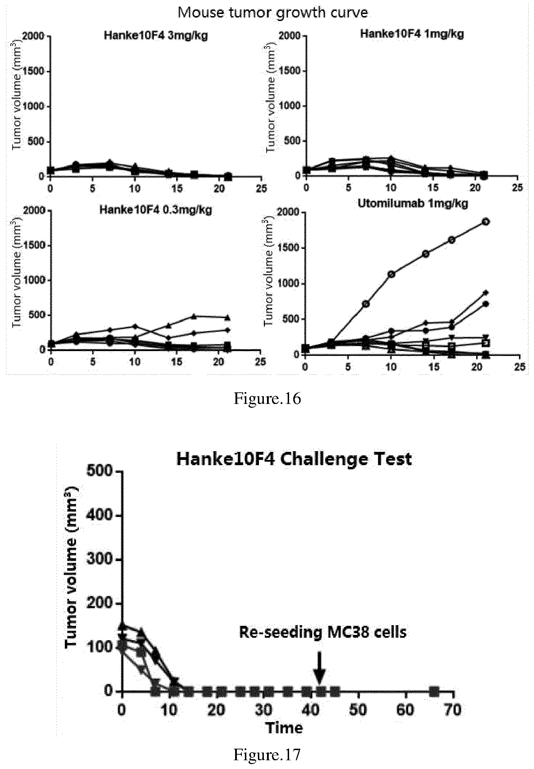

Through the above steps, the experiment proved that antibody Hanke10F4 could inhibit the growth of MC38 tumor, with obvious dose dependence, and the tumor suppression ability was significantly stronger than the anti-tumor efficacy of antibody Utomilumab under the same dosage. The specific results are shown in Figure 16.

In the Hanke10F4 administration group, after the tumor regressed, the observation was continued for 1 month, and the tumor did not recur. Mice whose tumors regressed were again inoculated with 4 × 10⁶ MC38 cells. After 15 days, tumors did not form, as shown in Figure 17, which shows that Hanke10F4 can not only inhibit tumor growth, but also mobilize the function of memory T cells in mice, with immune function against tumors of the same type, so that the tumor will not recur.

### 3.8 Safety assessment

### 3.8.1 Mouse toxicology experiment

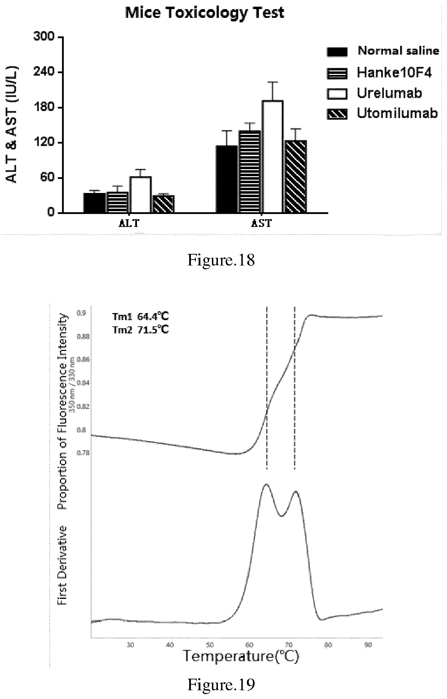

In the experiment, B-hCD137 (4-1BB) mice were selected to detect the anti-tumor efficacy of Hanke10F4 in vivo. The B-hCD137 (4-1BB) mouse model is a genetically engineered mouse with a human-derived h4-1BB gene that is chimeric in the genetic background, genome of C57BL/6 mouse, from Biocytogen Pharmaceuticals (Beijing) Co., Ltd, China. The test mice were randomly divided into 4 groups, and they were given physiological saline, Hanke10F4, Utomilumab and Urelumab, respectively, twice a week for 3 weeks, and the concentration of each administration was 30 mg/kg. After the administration, the tails of the mice were cut off and the blood was collected to detect the content of AST (aspartate aminotransferase) and ALT (alanine aminotransferase) in the peripheral blood. As shown in Figure 18, compared to the normal saline group, Urelumab group had a significantly higher AST content, indicating that the antibody has a risk of liver toxicity; while the AST and ALT contents of Hanke10F4 and Utomilumab groups were not significantly different from those of the normal saline group, indicating that Hanke10F4 and Utomilumab also have low toxicity.

### 3.8.2 Functional Observation Combination (FOB) test of intravenous injection of Hanke10F4 to SD rats

Hanke10F4 was administered to 40 SD rats (half female and half male) by a single intravenous injection, with three doses of high (30mg/kg), medium (10mg/kg), and low (3mg/kg). According to the functional observation combination (FOB) test at different time points, the effect of Hanke10F4 on the central nervous system of SD rats was evaluated.

During the experiment, in the placebo control group, Hanke10F4 low-dose group, Hanke10F4 medium-dose group, and Hanke10F4 high-dose group, there were no obvious abnormalities in the general state observation of rats. Before administration and 0.5 h, 6 h, 24 h, 48 h, and 168 h after administration, there were no drug-related abnormal changes in the body postures, hair erecting and other indicators of the animals in each group. No animals injured themselves or attacked other animals in the cage, and no animals died; The animals in each group showed no abnormal changes in indicators such as the difficulty of getting out of the cage, freehand restraint observation, tearing, salivation, secretion, exophthalmos, pupils, eyelid closure, skin color, hair erecting, breathing, etc. The animals in each group showed no abnormal changes in indicators such as approach reaction, contact reaction, panic reaction, abdominal tension, body tension, auricle reflex, tail pinching reflex observation, plane righting reflex observation, aerial righting reflex, pupil reflex observation, eyelid reflex, flexor response, extensor response, etc. In summary, under the experimental conditions, a single intravenous injection of 3, 10 and 30 mg/kg of Hanke10F4 in SD rats had no significant effect on the function of the animal's central nervous system.

### 3.8.3 Cynomolgus monkey toxicity

Four cynomolgus monkeys were randomly divided into two groups according to their gender and weight: a dose-climbing group and a fixed-dose group, 2 cynomolgus monkeys for each group, half female and half male. They were administered by intravenous injection. See Table 16 for details.

**Table 16. Cynomolgus monkey toxicity test groups and dosing regimens for each group**

| Group | Testing sample | Dosing cycle | Dosage (mg/kg) | Dosing concentration | Animal number | |
|---|---|---|---|---|---|---|
| | | | | (mg/mL) | Female | Male |
| Dose-climbing group | HankelOF4 | Week 1 | 3 | 0.3 | IF | 1M |
| | | Week 2 | 10 | 1.0 | | |
| | | Week 3 | 30 | 3.0 | | |
| | | Week 4 | 100 | 10.0 | | |
| | | Week 5 | 200 | 10.0 | | |
| Fixed-dose group | HankelOF4 | 1st ~ 5th week | 30 | 3.0 | 2F | 2M |

| | | | | | | |
|---|---|---|---|---|---|---|
| Note: The first digit of the animal number represents the group (1 and 2 respectively represent HankelOF4 dose-climbing group and HankelOF4 high-dose group); the second letter represents gender (F is female, M is male). | | | | | | |

The detection time points were once during the adaptation period, and once every 24 h after administration on D8, D15, D22, and D29; once at the end of the administration period (D35). The experimental results showed that during the administration period, the monkeys in each group had no abnormalities in general observation, body weight, food intake/food consumption, body temperature, respiration, electrocardiogram, blood pressure, blood coagulation index, etc. Compared with before administration, various indexes (alanine aminotransferase ALT, aspartate aminotransferase AST) of all the animal blood biochemical indexes of each Hanke10F4 dose group fluctuated within the normal range, and there was no obvious trend of change, see Table 17 for details. In the dose-climbing group, at a dose of ≥ 100 mg/kg, white blood cells, neutrophils, and monocytes in male animal increased, the cytokine IL-6 increased slightly, and then dropped back to the original level after re-administration, see table 18 for details. Therefore, the maximum tolerated dose (MTD) of Hanke10F4 in cynomolgus monkeys was ≥200 mg/kg.

**Table 17. Individual data of cynomolgus monkey hematology**

| Animal number | Test date | RBC red blood cells (×10^12/L) | HGB hemoglobin (g/L) | WBC white blood cells (×10^12/L) | ALT (U/L) | AST (U/L) |
|---|---|---|---|---|---|---|
| IF | D4 | 5.32 | 130 | 4.96 | 64.0 | 49.8 |
| 2F | D4 | 5.94 | 135 | 12.74 | 49.0 | 37.3 |
| IF | D9 | 4.89 | 119 | 5.25 | 77.1 | 83.2 |
| 2F | D9 | 5.92 | 136 | 12.68 | 32.7 | 34.1 |
| IF | D16 | 5.04 | 125 | 3.77 | 53.1 | 66.6 |
| 2F | D16 | 6.13 | 140 | 11.47 | 31.3 | 35.7 |
| IF | D23 | 4.53 | 113 | 5.04 | 46.4 | 52.5 |
| 2F | D23 | 5.69 | 130 | 13.33 | 34.8 | 44.6 |
| IF | D30 | 5.09 | 129 | 5.85 | 59.7 | 91.3 |
| 2F | D30 | 5.99 | 135 | 10.55 | 40.1 | 54.1 |
| IF | D35 | 5.35 | 133 | 5.15 | 41.3 | 42.2 |
| 2F | D35 | 5.95 | 134 | 8.04 | 31.2 | 34.1 |

**Table 18. Individual data of cynomolgus monkey cytokines**

| Animal number | Test phase | IL-6(pg/ml) | IP-10(pg/ml) | IFN-γ(pg/ml ) |
|---|---|---|---|---|
| IF | D4 | 0.28 | 0.00 | 0.00 |
| IF | D9 | 0.00 | 0.00 | 0.00 |
| IF | D16 | 1.33 | 0.00 | 0.00 |
| IF | D23 | 2.60 | 0.00 | 0.00 |
| IF | D30 | 1.48 | 0.00 | 0.00 |
| 2F | D4 | 0.00 | 0.00 | 0.00 |
| 2F | D9 | 0.01 | 0.00 | 0.00 |
| 2F | D16 | 0.01 | 0.00 | 0.00 |
| 2F | D23 | 0.00 | 0.00 | 0.00 |
| 2F | D30 | 0.00 | 0.00 | 0.00 |
| 2F | D35 | 0.00 | 0.00 | 0.00 |
| IF | D35 | 0.00 | 0.00 | 0.00 |
| 1M | D4 | 0.70 | 0.00 | 0.00 |
| 1M | D9 | 0.00 | 0.00 | 0.00 |
| 1M | D16 | 0.70 | 0.00 | 0.00 |
| 1M | D23 | 28.63 | 0.00 | 0.00 |
| 1M | D30 | 28.94 | 0.00 | 0.00 |
| 1M | D35 | 0.00 | 0.00 | 0.00 |
| 2M | D4 | 0.00 | 0.00 | 0.00 |
| 2M | D9 | 0.00 | 0.00 | 0.00 |
| 2M | D16 | 0.80 | 0.00 | 0.00 |
| 2M | D23 | 0.52 | 0.00 | 0.00 |
| 2M | D30 | 0.00 | 0.00 | 0.00 |
| 2M | D35 | 0.00 | 0.00 | 0.00 |

### 3.9 Stability

### (1) Accelerated stability

Two buffers were prepared, F01 (pH5.2) and F02 (pH5.5), Hanke10F4 was set to 20mg/ml, and placed at 4°C for 3 months (3M) to detect various physical and chemical properties such as antibody concentration, aggregation and degradation, etc., including: detection of the Hanke10F4 protein concentration by a variable optical path spectrophotometer, detection of Hanke10F4 molecular size, heteroplasmic purity (SEC-HPLC) and charge heterogeneity purity (CEX-HPLC) by high performance liquid chromatography, and detection of Hanke10F4 non-reducing capillary gel electrophoresis protein purity (NR-CE) by capillary electrophoresis analysis system. The results are shown in Table 19, Hanke10F4 was placed in different pH buffers at 4°C for 3 months, compared with the sample of initial time (Hanke10F4/T0), there was no significant change, and Hanke10F4 had good stability.

At the same time, the Hanke10F4 sample and the sample at the beginning time of the experiment (Hanke10F4/T0) were taken and stored at 4°C for 1 month (1M), 2 months (2M), and 3 months (3M). The biological activity of the antibodies was detected by the reporter gene method described above, the highest concentration was set to 4ug/ml, and 9 concentration points were obtained by diluting with a 2-fold gradient. The results of the experiment are shown in Figure 13B. The biological activity of Hanke10F4 was good after being stored at 4°C for 3 months.

**Table 19. HankelOF4 accelerated stability test**

| Test items | Protein concentration | | | | | | SEC-HPLC | | | | | |
|---|---|---|---|---|---|---|---|---|---|---|---|---|
| Number | mg/ml | | | | | | Aggregate peak% | | Main peak% | | Degradation peak% | |
| | TO | | | 3M | | | TO | 3M | TO | 3M | TO | 3M |
| F01 | 20.0 | | | 19.8 | | | 0.2 | 0.4 | 99.7 | 99.5 | 0 | 0.1 |
| F02 | 20.1 | | | 19.9 | | | 0.3 | 0.5 | 99.7 | 99.4 | 0.1 | 0.1 |

| Test items | CEX-HPLC | | | | | | NR-CE | | | | | |
|---|---|---|---|---|---|---|---|---|---|---|---|---|
| Number | Acidic peak | | Main peak | | Base peak | | High molecular weight substance% | | Main peak | | Other% | |
| | TO | 3M | TO | 3M | TO | 3M | TO | 3M | TO | 3M | TO | 3M |
| F01 | 10.5 | 11 | 82.1 | 82.1 | 7.4 | 6.9 | 0.2 | 0.2 | 99.5 | 99.2 | 0.3 | 0.5 |
| F02 | 10.2 | 10.9 | 82.3 | 82.3 | 7.4 | 6.8 | 0.1 | 0.2 | 99.4 | 99.3 | 0.5 | 0.5 |

| | | | | | | | | | | | | |
|---|---|---|---|---|---|---|---|---|---|---|---|---|
| Note: TO is the initial time; 3M is the end of 3 months. | | | | | | | | | | | | |

### (2) Tm value detection

The fluorescence intensity of the antibody Hanke10F4 at two wavelengths (350 nm and 330 nm) were detected by differential scanning fluorimetry (DSF), and the fluorescence intensity ratio was calculated. The changes of fluorescence intensity and fluorescence peak during the protein unfolding process were detected to obtain the temperature at which half of the Hanke10F4 protein unfolds, that is the Tm value. The results showed the Tm1 value of Hanke10F4 was 64.4°C, and the Tm2 value was 71.5°C (Tm1 and Tm2 are the two temperatures at which the fluorescence peak changes during protein unfolding), that was, the stability of the antibody Hanke10F4 was good (Figure 19).

### Industrial application

The antibody Hanke10F4 provided by the present invention can bind to human 4-1BB, exhibit high affinity to human 4-1BB and effectively enhance T cell response. On the one hand, the antibody competitively binds to human 4-1BB with human 4-1BBL to a certain extent; on the other hand, the antibody induces the production of INF- γ in the T cell costimulation assay, and can significantly inhibit tumor growth in a mouse model, and the tumor will not recur. The antibody of the present invention can be used for regulating T cell and antibody-mediated immune response, and can be used for anti-tumor immunotherapy. The fusion antibody of the single-chain antibody provided by the present invention can bind to human and monkey 4-1BB.

The binding of the antibodies of the present invention to human 4-1BB leads to an enhanced immune response. The antibodies and their antigen-binding fragments provided by the present invention can be used as immune enhancers or immune modulators for T cell-mediated autoimmune diseases. The antibodies and their antigen-binding fragments can also be used as diagnostic tools to detect 4-1BB in the blood or tissues of patients with cancer, autoimmune or other diseases.

The antibodies provided by the present invention have good anti-tumor efficacy, strong safety and good stability.

The antibodies and their antigen-binding fragments provided by the present invention have a wide range of therapeutic applications as immune modulators in diseases such as cancer, autoimmune diseases, inflammatory diseases, and infectious diseases, etc.

## Claims

1. An antibody, **characterized in that**: the amino acid sequences of HCDR1, HCDR2 and HCDR3 in the heavy chain variable region of the antibody are shown sequentially at positions 31-35, positions 50-64, and positions 98-106 in SEQ ID No. 1 from the N-terminus.

2. The antibody according to claim 1, **characterized in that**: the amino acid sequences of LCDR1, LCDR2 and LHCDR3 in the light chain variable region of the antibody are shown sequentially at positions 24-34, positions 50-56, and positions 89-97 in SEQ ID No. 2 from the N-terminus.

3. The antibody according to claim 1, **characterized in that**: the amino acid sequence of the variable region of the heavy chain is any of the following:
(a1) Positions 1-117 in SEQ ID No.1 from the N-terminus, or with more than 99%, more than 95%, more than 90%, more than 85%, more than 80% or more than 75% of consistency with positions 1-117 in SEQ ID No.1 from the N-terminus;
(a2) Positions 1-117 in SEQ ID No.18 from the N-terminus, or with more than 99%, more than 95%, more than 90%, more than 85%, more than 80% or more than 75% of consistency with positions 1-117 in SEQ ID No.18 from the N-terminus;
(a3) Positions 1-117 in SEQ ID No.19 from the N-terminus, or with more than 99%, more than 95%, more than 90%, more than 85%, more than 80% or more than 75% of consistency with positions 1-117 in SEQ ID No.19 from the N-terminus;
(a4) Positions 1-117 in SEQ ID No.20 from the N-terminus, or with more than 99%, more than 95%, more than 90%, more than 85%, more than 80% or more than 75% of consistency with positions 1-117 in SEQ ID No.20 from the N-terminus;
(a5) Positions 1-117 in SEQ ID No.21 from the N-terminus, or with more than 99%, more than 95%, more than 90%, more than 85%, more than 80% or more than 75% of consistency with positions 1-117 in SEQ ID No.21 from the N-terminus;
(a6) Positions 1-117 in SEQ ID No.22 from the N-terminus, or with more than 99%, more than 95%, more than 90%, more than 85%, more than 80% or more than 75% of consistency with positions 1-117 in SEQ ID No.22 from the N-terminus;
(a7) Positions 1-117 in SEQ ID No.23 from the N-terminus, or with more than 99%, more than 95%, more than 90%, more than 85%, more than 80% or more than 75% of consistency with positions 1-117 in SEQ ID No.23 from the N-terminus;
(a8) Positions 1-117 in SEQ ID No.24 from the N-terminus, or with more than 99%, more than 95%, more than 90%, more than 85%, more than 80% or more than 75% of consistency with positions 1-117 in SEQ ID No.24 from the N-terminus;
(a9) Positions 1-117 in SEQ ID No.25 from the N-terminus, or with more than 99%, more than 95%, more than 90%, more than 85%, more than 80% or more than 75% of consistency with positions 1-117 in SEQ ID No.25 from the N-terminus;
(a10) Positions 1-117 in SEQ ID No.26 from the N-terminus, or with more than 99%, more than 95%, more than 90%, more than 85%, more than 80% or more than 75% of consistency with positions 1-117 in SEQ ID No.26 from the N-terminus;
(a11) Positions 1-117 in SEQ ID No.27 from the N-terminus, or with more than 99%, more than 95%, more than 90%, more than 85%, more than 80% or more than 75% of consistency with positions 1-117 in SEQ ID No.27 from the N-terminus;
(a12) Positions 1-117 in SEQ ID No.28 from the N-terminus, or with more than 99%, more than 95%, more than 90%, more than 85%, more than 80% or more than 75% of consistency with positions 1-117 in SEQ ID No.28 from the N-terminus.

4. The antibody according to claim 2, **characterized in that**: the amino acid sequence of the variable region of the light chain is any of the following:
(b1) Positions 1-107 in SEQ ID No.2 from the N-terminus, or with more than 99%, more than 95%, more than 90%, more than 85%, more than 80% or more than 75% of consistency with positions 1-107 in SEQ ID No.2 from the N-terminus;
(b2) Positions 133-239 in SEQ ID No.18 from the N-terminus, or with more than 99%, more than 95%, more than 90%, more than 85%, more than 80% or more than 75% of consistency with positions 133-239 in SEQ ID No.18 from the N-terminus;
(b3) Positions 133-239 in SEQ ID No.19 from the N-terminus, or with more than 99%, more than 95%, more than 90%, more than 85%, more than 80% or more than 75% of consistency with positions 133-239 in SEQ ID No.19 from the N-terminus;
(b4) Positions 133-239 in SEQ ID No.20 from the N-terminus, or with more than 99%, more than 95%, more than 90%, more than 85%, more than 80% or more than 75% of consistency with positions 133-239 in SEQ ID No.20 from the N-terminus;
(b5) Positions 133-239 in SEQ ID No.21 from the N-terminus, or with more than 99%, more than 95%, more than 90%, more than 85%, more than 80% or more than 75% of consistency with positions 133-239 in SEQ ID No.21 from the N-terminus;
(b6) Positions 133-239 in SEQ ID No.22 from the N-terminus, or with more than 99%, more than 95%, more than 90%, more than 85%, more than 80% or more than 75% of consistency with positions 133-239 in SEQ ID No.22 from the N-terminus;
(b7) Positions 133-239 in SEQ ID No.23 from the N-terminus, or with more than 99%, more than 95%, more than 90%, more than 85%, more than 80% or more than 75% of consistency with positions 133-239 in SEQ ID No.23 from the N-terminus;
(b8) Positions 133-239 in SEQ ID No.24 from the N-terminus, or with more than 99%, more than 95%, more than 90%, more than 85%, more than 80% or more than 75% of consistency with positions 133-239 in SEQ ID No.24 from the N-terminus;
(b9) Positions 133-239 in SEQ ID No.25 from the N-terminus, or with more than 99%, more than 95%, more than 90%, more than 85%, more than 80% or more than 75% of consistency with positions 133-239 in SEQ ID No.25 from the N-terminus;
(b10) Positions 133-239 in SEQ ID No.26 from the N-terminus, or with more than 99%, more than 95%, more than 90%, more than 85%, more than 80% or more than 75% of consistency with positions 133-239 in SEQ ID No.26 from the N-terminus;
(b11) Positions 133-239 in SEQ ID No.27 from the N-terminus, or with more than 99%, more than 95%, more than 90%, more than 85%, more than 80% or more than 75% of consistency with positions 133-239 in SEQ ID No.27 from the N-terminus;
(b12) Positions 133-239 in SEQ ID No.28 from the N-terminus, or with more than 99%, more than 95%, more than 90%, more than 85%, more than 80% or more than 75% of consistency with positions 133-239 in SEQ ID No.28 from the N-terminus.

5. The antibody of any one according to claims 1-4, **characterized in that**: the antibody is IgG, IgM, IgE, IgA or IgD.

6. The antibody according to claims 5, **characterized in that**: the IgG is IgG1, IgG2, IgG3 or IgG4 subtype.

7. The antibody of any one according to claims 1-6, **characterized in that**: the light chain type of the antibody is kappa chain or lambda chain.

8. The antibody according to claims 6 or 7, **characterized in that**: the antibody is IgG4 subtype, and the light chain type of the antibody is Kappa chain.

9. The antibody of any one according to claims 1-8, **characterized in that**: the antibody is a humanized antibody.

10. The antibody of any one according to claims 1-9, **characterized in that**: the amino acid sequence of the heavy chain of the antibody is SEQ ID No. 1, or with more than 99%, more than 95%, more than 90%, and more than 85%, more than 80% or more than 75% of consistency with SEQ ID No. 1.

11. The antibody of any one according to claims 1-10, **characterized in that**:
the amino acid sequence of the light chain of the antibody is SEQ ID No. 2, or with more than 99%, more than 95%, more than 90%, and more than 85%, more than 80% or more than 75% of consistency with SEQ ID No.2.

12. Antigen-binding fragments of said antibody of any one of claims 1-11.

13. The antigen-binding fragment according to claims 12, **characterized in that**:
the antigen-binding fragment includes one or more of the following: the light chain of the antibody; the heavy chain of the antibody; the variable region of the light chain of the antibody; the variable region of the heavy chain of the antibody; one or more CDR regions of the antibody.

14. The antigen-binding fragment according to claims 13, **characterized in that**:
the antigen-binding fragment is any one of the following: Fab fragment; F(ab')2 fragment; Fd fragment; FV fragment; isolated CDR; single-chain antibody.

15. The antigen-binding fragment according to claims 14, **characterized in that**:
the antigen-binding fragment is a single-chain antibody; the complete amino acid sequence of the single-chain antibody is any one of the following:
(c1) Positions 1-239 of SEQ ID No.16, or with more than 99%, more than 95%, more than 90%, more than 85%, more than 80% or more than 75% of consistency with positions 1-239 of SEQ ID No.16;
(c2) Positions 1-239 of SEQ ID No.18, or with more than 99%, more than 95%, more than 90%, more than 85%, more than 80% or more than 75% of consistency with positions 1-239 of SEQ ID No.18;
(c3) Positions 1-239 of SEQ ID No.19, or with more than 99%, more than 95%, more than 90%, more than 85%, more than 80% or more than 75% of consistency with positions 1-239 of SEQ ID No.19;
(c4) Positions 1-239 of SEQ ID No.20, or with more than 99%, more than 95%, more than 90%, more than 85%, more than 80% or more than 75% of consistency with positions 1-239 of SEQ ID No.20;
(c5) Positions 1-239 of SEQ ID No.21, or with more than 99%, more than 95%, more than 90%, more than 85%, more than 80% or more than 75% of consistency with positions 1-239 of SEQ ID No.21;
(c6) Positions 1-239 of SEQ ID No.22, or with more than 99%, more than 95%, more than 90%, more than 85%, more than 80% or more than 75% of consistency with positions 1-239 of SEQ ID No.22;
(c7) Positions 1-239 of SEQ ID No.23, or with more than 99%, more than 95%, more than 90%, more than 85%, more than 80% or more than 75% of consistency with positions 1-239 of SEQ ID No.23;
(c8) Positions 1-239 of SEQ ID No.24, or with more than 99%, more than 95%, more than 90%, more than 85%, more than 80% or more than 75% of consistency with positions 1-239 of SEQ ID No.24;
(c9) Positions 1-239 of SEQ ID No.25, or with more than 99%, more than 95%, more than 90%, more than 85%, more than 80% or more than 75% of consistency with positions 1-239 of SEQ ID No.25;
(c10) Positions 1-239 of SEQ ID No.26, or with more than 99%, more than 95%, more than 90%, more than 85%, more than 80% or more than 75% of consistency with positions 1-239 of SEQ ID No.26;
(c11) Positions 1-239 of SEQ ID No.27, or with more than 99%, more than 95%, more than 90%, more than 85%, more than 80% or more than 75% of consistency with positions 1-239 of SEQ ID No.27;
(c12) Positions 1-239 of SEQ ID No.28, or with more than 99%, more than 95%, more than 90%, more than 85%, more than 80% or more than 75% of consistency with positions 1-239 of SEQ ID No.28 (the inconsistency is preferably in the framework region (FR)).

16. An antibody fusion protein formed by said antibody of any one of claims 1-11 or said antigen-binding fragment of any one of claims 12-15 and other proteins.

17. The antibody fusion protein according to claim 16, **characterized in that**: the antibody fusion protein is a single-chain antibody with a tag protein formed by said single-chain antibody of claim 14 or 15 and a tag protein.

18. The antibody fusion protein according to claim 17, **characterized in that**: the tag protein is Fc.

19. The antibody fusion protein according to claim 18, **characterized in that**: the Fc is IgG Fc.

20. The antibody fusion protein according to claim 18, **characterized in that**: the IgG is IgG 4.

21. The antibody fusion protein of any one according to claim 18-20, **characterized in that**: the amino acid sequence of the Fc is shown at positions 240-472 of SEQ ID No. 16, or with with more than 99%, more than 95%, more than 90%, and more than 85%, more than 80% or more than 75% of consistency with positions 240-472 in SEQ ID No.16 from the N-terminus.

22. The antibody fusion protein according to claim 21, **characterized in that**: the amino acid sequence of said single-chain antibody with the tag protein is any one of the following:
(d1) As shown in SEQ ID No.16, or with more than 99%, more than 95%, more than 90%, more than 85%, more than 80% or more than 75% of consistency with SEQ ID No.16;
(d2) As shown in SEQ ID No.18, or with more than 99%, more than 95%, more than 90%, more than 85%, more than 80% or more than 75% of consistency with SEQ ID No.18;
(d3) As shown in SEQ ID No.19, or with more than 99%, more than 95%, more than 90%, more than 85%, more than 80% or more than 75% of consistency with SEQ ID No.19;
(d4) As shown in SEQ ID No.20, or with more than 99%, more than 95%, more than 90%, more than 85%, more than 80% or more than 75% of consistency with SEQ ID No.20;
(d5) As shown in SEQ ID No.21, or with more than 99%, more than 95%, more than 90%, more than 85%, more than 80% or more than 75% of consistency with SEQ ID No.21;
(d6) As shown in SEQ ID No.22, or with more than 99%, more than 95%, more than 90%, more than 85%, more than 80% or more than 75% of consistency with SEQ ID No.22;
(d7) As shown in SEQ ID No.23, or with more than 99%, more than 95%, more than 90%, more than 85%, more than 80% or more than 75% of consistency with SEQ ID No.23;
(d8) As shown in SEQ ID No.24, or with more than 99%, more than 95%, more than 90%, more than 85%, more than 80% or more than 75% of consistency with SEQ ID No.24;
(d9) As shown in SEQ ID No.25, or with more than 99%, more than 95%, more than 90%, more than 85%, more than 80% or more than 75% of consistency with SEQ ID No.25;
(d10) As shown in SEQ ID No.26, or with more than 99%, more than 95%, more than 90%, more than 85%, more than 80% or more than 75% of consistency with SEQ ID No.26;
(d11) As shown in SEQ ID No.27, or with more than 99%, more than 95%, more than 90%, more than 85%, more than 80% or more than 75% of consistency with SEQ ID No.27;
(d12) As shown in SEQ ID No.28, or with more than 99%, more than 95%, more than 90%, more than 85%, more than 80% or more than 75% of consistency with SEQ ID No.28.

23. A double antibody formed by the antibody of any one of claims 1-11 or the antigen-binding fragment of any one of claims 12-15 and antibodies of other targets.

24. A nucleic acid molecule, **characterized in that**: the nucleic acid molecule encodes the antibody of any one of claims 1-11, or the antigen-binding fragment of any one of claims 12-15, or the antibody fusion protein of any one of claims 16-22, or the double antibody of claim 23.

25. The nucleic acid molecule according to claim 23, **characterized in that**: in the nucleic acid molecule, the nucleotide sequences encoding HCDR1, HCDR2 and HCDR3 in the heavy chain variable region of the antibody of any one of claims 1-11 are at positions 91-105, positions 148-192, and positions 292-318 sequentially in SEQ ID No.3 from the 5'end.

26. The nucleic acid molecule according to claim 23, **characterized in that**: in the nucleic acid molecule, the nucleotide sequences encoding LCDR1, LCDR2 and LHCDR3 in the light chain variable region of the antibody of any one of claims 1-11 are at positions 70-102, positions 148-168, and positions 265-291 sequentially in SEQ ID No.4 from the 5'end.

27. The nucleic acid molecule according to claim 25, **characterized in that**: in the nucleic acid molecule, the nucleotide sequence encoding the heavy chain variable region of the antibody is at positions 1-351 in SEQ ID No.3 from the 5'end, or with more than 99%, more than 95%, more than 90%, more than 85%, more than 80% or more than 75% of consistency with positions 1-351 in SEQ ID No.3 from the 5'end.

28. The nucleic acid molecule according to claim 26, **characterized in that**: the nucleotide sequence encoding the light chain variable region of the antibody is at positions 1-321 in SEQ ID No.4 from the 5'end, or with more than 99%, more than 95%, more than 90%, more than 85%, more than 80% or more than 75% of consistency with positions 1-321 in SEQ ID No.4 from the 5'end.

29. The nucleic acid molecule according to claim 27, **characterized in that**: in the nucleic acid molecule, the nucleotide sequence encoding the heavy chain variable region of the antibody is SEQ ID No.3, or with more than 99%, more than 95%, more than 90%, more than 85%, more than 80% or more than 75% of consistency with SEQ ID No.3.

30. The nucleic acid molecule according to claim 28, **characterized in that**: in the nucleic acid molecule, the nucleotide sequence encoding the light chain variable region of the antibody is SEQ ID No.4, or with more than 99%, more than 95%, more than 90%, more than 85%, more than 80% or more than 75% of consistency with SEQ ID No.4.

31. The nucleic acid molecule according to claim 27 or 28, **characterized in that**: the nucleotide sequence encoding the single-chain antibody is at positions 1-717 in SEQ ID No.17 from the 5'end, or with more than 99%, more than 95%, more than 90%, more than 85%, more than 80% or more than 75% of consistency with positions 1-717 in SEQ ID No.17 from the 5'end.

32. The nucleic acid molecule according to claim 31, **characterized in that**: the nucleotide sequence encoding the single-chain antibody with the tag protein is SEQ ID No.17, or with more than 99%, more than 95%, more than 90%, more than 85%, more than 80% or more than 75% of consistency with SEQ ID No.17.

33. An expression cassette, recombinant vector or recombinant cell containing the nucleic acid molecule of any one of claims 24-32.

34. The expression cassette, recombinant vector or recombinant cell according to claim 33, **characterized in that**: the recombinant cell is a higher eukaryotic host cell, a lower eukaryotic host cell, or a prokaryotic cell.

35. The expression cassette, recombinant vector or recombinant cell according to claim 34, **characterized in that**: the higher eukaryotic host cell is a mammalian cell; the lower eukaryotic host cell is a yeast cell; the prokaryotic cell is a bacterial cell or Escherichia coli.

36. The expression cassette, recombinant vector or recombinant cell according to claim 33, **characterized in that**: the recombinant vector is a plasmid, cosmid, phage or virus vector.

37. An ADC antibody, **characterized in that**: the ADC antibody is formed by the antibody of any one of claims 1-11, or the antigen-binding fragment of any one of claims 12-15, or the antibody fusion protein of any one of claims 16-22, or the double antibody of claim 23 conjugated with small molecule drug with biological activity.

38. A pharmaceutical composition, **characterized in that**: the pharmaceutical composition comprises:
(A1) The antibody of any one of claims 1-11 or the antigen-binding fragment of any one of claims 12-15 or the antibody fusion protein of any one of claims 16-22 or the double antibody of claim 23 or the ADC antibody of claim 37;
(A2) Pharmaceutically acceptable excipients, diluents or carriers.

39. Application of the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 in any of the following:
(B1) Preparation of products for testing 4-1BB, or application in testing 4-1BB;
(B2) Preparation of products for blocking the 4-1BB/4-1BBL signal pathway, or application in blocking the 4-1BB/4-1BBL signal pathway;
(B3) Preparation of products for stimulating T cell activation, or application in stimulating T cell activation;
(B4) Preparation of products for promoting the secretion of IFN-γ by T cells, or application in promoting the secretion of IFN-γ by T cells;
(B5) Preparation of products for inhibiting the growth of colon cancer cells, or application in inhibiting the growth of colon cancer cells;
(B6) Preparation of products for inhibiting the growth of colon cancer tumors, or application in inhibiting the growth of colon cancer tumors;
(B7) Preparation of products for treating and/or preventing colon cancer, or application in treating and/or preventing colon cancer;
(B8) Using as an immune enhancer, or application in preparing an immune enhancer;
(B9) Using an immune modulator, or application in preparing an immune modulator;
(B10) Preparation of products for the treatment and/or prevention and/or diagnosis of diseases **characterized by** 4-1BB expression dysregulation, or application in the treatment and/or prevention and/or diagnosis of diseases **characterized by** 4-1BB expression dysregulation.
(B11) Preparation of products for the treatment and/or prevention and/or diagnosis of cancer, or application in the treatment and/or prevention and/or diagnosis of cancer;
(B12) Preparation of products for the treatment and/or prevention and/or diagnosis of autoimmune diseases, or application in treatment and/or prevention and/or diagnosis of autoimmune diseases;
(B13) Preparation of products for the treatment and/or prevention and/or diagnosis of inflammatory diseases, or application in the treatment and/or prevention and/or diagnosis of inflammatory diseases;
(B14) Preparation of products for the treatment and/or prevention and/or diagnosis of infectious diseases, or application in the treatment and/or prevention and/or diagnosis of infectious diseases.

40. The application according to claim 39, **characterized in that**: the 4-1BB is human 4-1BB or monkey 4-1BB.

41. The application according to claim 39, **characterized in that**: the cancer is a cancer in which the expression of 4-1BB is dysregulated; the autoimmune disease is an autoimmune disease in which the expression of 4-1BB is dysregulated; the inflammatory disease is an inflammatory disease in which the expression of 4-1BB is dysregulated; the infectious disease is an infectious disease in which the expression of 4-1BB is dysregulated.

42. Any of the following methods:
(C1) A method for detecting 4-1BB, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 to detect the sample to be tested;
(C2) A method for blocking the 4-1BB/4-1BBL signal pathway, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to block the 4-1BB/4-1BBL signal pathway;
(C3) A method for stimulating T cell activation, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to stimulate T cell activation;
(C4) A method for promoting the secretion of IFN-γ by T cells, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to promote the secretion of IFN-γ by T cells;
(C5) A method for inhibiting the growth of colon cancer cells, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to inhibit the growth of colon cancer cells;
(C6) A method for inhibiting the growth of colon cancer tumors, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to inhibit the growth of colon cancer tumors;
(C7) A method for treating and/or preventing colon cancer, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to treat and/or prevent colon cancer;
(C8) A method for preparing an immune enhancer, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 as an active ingredient to prepare an immune enhancer;
(C9) A method for preparing an immune modulator, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 as an active ingredient to prepare an immune modulator;
(C10) A method for treating and/or preventing and/or diagnosing a disease **characterized by** 4-1BB expression dysregulation, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to treat and/or prevent and/or diagnose a disease **characterized by** 4-1BB expression dysregulation;
(C11) A method for treating and/or preventing and/or diagnosing cancer, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to treat and/or prevent and/or diagnose cancer;
(C12) A method for treating and/or preventing and/or diagnosing autoimmune diseases, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to treat and/or prevent and/or diagnose autoimmune diseases;
(C13) A method for treating and/or preventing and/or diagnosing inflammatory diseases, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to treat and/or prevent and/or diagnose inflammatory disease;
(C14) A method for treating and/or preventing and/or diagnosing infectious diseases, comprising the following steps: using the antibody of any one of claims 1-11 or the antigen-binding fragment of any one of claims 12-15 or the antibody fusion protein of any one of claims 16-22 or the double antibody of claim 23 or the nucleic acid molecule of any one of claims 24-32 or the expression cassette, recombinant vector or recombinant cell of any one of claims 33-36 or the ADC antibody of claim 37 or the pharmaceutical composition of claim 38 to treat and/or prevent and/or diagnose infectious diseases;

43. The method according to claim 42, **characterized in that**: the 4-1BB is human 4-1BB or monkey 4-1BB.

44. The method according to claim 42, **characterized in that**: the cancer is a cancer in which the expression of 4-1BB is dysregulated; the autoimmune disease is an autoimmune disease in which the expression of 4-1BB is dysregulated; the inflammatory disease is an inflammatory disease in which the expression of 4-1BB is dysregulated; the infectious disease is an infectious disease in which the expression of 4-1BB is dysregulated.
